# EUROPEAN PATENT SPECIFICATION

(11) **EP 4 329 735 B1**
(45) Date of publication and mention of the grant of the patent: **25.02.2026**
(21) Application number: 22796393.1
(22) Date of filing: 15.04.2022
(51) Int. Cl.: C07D 413/04, A61K 31/015, A61K 31/16, A61K 31/4192, A61P 25/00

(54) **KCNT1 INHIBITORS AND METHODS OF USE**
KCNT1-INHIBITOREN UND VERFAHREN ZUR VERWENDUNG
INHIBITEURS DE KCNT1 ET PROCÉDÉS D'UTILISATION

(30) Priority: 29.04.2021 US 202163181528 P
(43) Date of publication of application: 06.03.2024
(73) Proprietor: Praxis Precision Medicines, Inc., Boston, MA 02110 (US)
(72) Inventor: GRIFFIN, Andrew Mark, Boston, MA 02110 (US); BOTELLA, Gabriel Martinez, Boston, MA 02110 (US); CHARIFSON, Paul S., Boston, MA 02110 (US)
(74) Representative: J A Kemp LLP
(86) International application number: PCT/US2022/025055
(87) International publication number: WO 2022/231873

(56) References cited:
- WO-A1-2020/227101
- WO-A1-2021/173930
- WO-A1-2022/211595
- WO-A1-98/57969
- WO-A2-2010/018458
- US-A- 4 160 829
- US-A1- 2020 247 793
- GRIFFIN ANDREW M., KAHLIG KRISTOPHER M., HATCH ROBERT JOHN, HUGHES ZOË A., CHAPMAN MARK L., ANTONIO BRETT, MARRON BRIAN E., WITTMA: "Discovery of the First Orally Available, Selective K Na 1.1 Inhibitor: In Vitro and In Vivo Activity of an Oxadiazole Series", ACS MEDICINAL CHEMISTRY LETTERS, AMERICAN CHEMICAL SOCIETY, US, vol. 12, no. 4, 8 April 2021 (2021-04-08), US , pages 593 - 602, XP093003438, ISSN: 1948-5875, DOI: 10.1021/acsmedchemlett.0c00675
- DATABASE PubChem COMPOUND 26 March 2005 (2005-03-26), ANONYMOUS : "COMPOUND SUMMARY Methyl isocyanate | CH3NCO ", XP093003443, Database accession no. CID 1222
- DATABASE Pubchem COMPOUND 5 December 2007 (2007-12-05), ANONYMOUS : "COMPOUND SUMMARY N-propyl-alpha-(3-ethyl-1,2,4-oxadiazol-5-yl)-propionamide | C10H17N3O2", XP093003446, Database accession no. CID 21312965

## Description

### CROSS-REFERENCE TO RELATED APPLICATION

This application claims the benefit of, and relies on the filing date of, U.S. provisional patent application number 63/181,528, filed 29 April 2021.

### FIELD OF THE DISCLOSURE

The present disclosure is generally directed to KCNT1 inhibitors comprising an oxadiazole core, as well as pharmaceutical compositions and such compounds for use in methods of treatment.

### BACKGROUND OF THE DISCLOSURE

Potassium sodium-activated channel subfamily T member 1 ("KCNT1") is one of the genes in a family of genes responsible for providing the instructions to make potassium channels. KCNT1 encodes sodium-activated potassium channels known as Slack (Sequence like a calcium-activated K⁺ channel). These channels are found in neurons throughout the brain and can mediate a sodium-activated potassium current *I*_{KNa}. This delayed outward current can regulate neuronal excitability and the rate of adaption in response to maintained stimulation. Abnormal Slack activity has been associated with development of early onset epilepsies and intellectual impairment. Accordingly, pharmaceutical compounds that selectively regulate sodium-activated potassium channels, e.g., abnormal KCNT1 or abnormal *I*_{KNa}, are useful in treating a neurological disease or disorder or a disease or condition related to excessive neuronal excitability and/or KCNT1 gain-of-function mutations.

### SUMMARY OF THE DISCLOSURE

Described herein are compounds and compositions useful for preventing and/or treating a disease, disorder, or condition, e.g., a neurological disorder, a disorder associated with excessive neuronal excitability, or a disorder associated with a gain-of-function mutation in a gene, for example, KCNT1. In the present description all references to methods for treatment as part of the invention are to be interpreted as being directed to compounds for use in such methods.

In one aspect, the present disclosure features a compound of Formula I having an oxadiazole core: 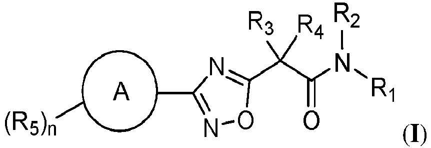 or a pharmaceutically acceptable salt thereof, wherein
ring A is a 5-6 membered heteroaryl;
R₁ is chosen from a C₁₋₆alkyl, a C₃₋₈cycloalkyl, a phenyl, a 3-7 membered heterocyclyl, or a 5-6 membered heteroaryl, wherein the C₁₋₆alkyl, C₃₋₈cycloalkyl, phenyl, 3-7 membered heterocyclyl, or 5-6 membered heteroaryl optionally comprises one or more R₆ substituents;
R₂ is chosen from hydrogen or a C₁₋₆alkyl; or
R₁ and R₂ are taken together with the nitrogen attached to R₁ and R₂ to form a 3-7 membered heterocyclyl optionally comprising one or more R₆;
R₃ is chosen from hydrogen or a C₁₋₆alkyl optionally comprising one or more substituents chosen from a halogen, a C₁₋₆haloalkyl, a C₁₋₆alkoxy, or a C₁₋₆haloalkoxy;
R₄ is a C₁₋₆alkyl optionally comprising one or more substituents chosen from a halogen, a C₁₋₆haloalkyl, a C₁₋₆alkoxy, or a C₁₋₆haloalkoxy; or
R₃ and R₄ are taken together with the carbon attached to R₃ and R₄ to form a C₃₋₈cycloalkylene or a 3-7 membered heterocycloalkylene;
R₅ is each independently chosen from a halogen, a C₁₋₆alkyl, a C₁₋₆haloalkyl, a C₁₋₆alkoxy, a C₁₋₆haloalkoxy, a N(R₇)₂, a C₁₋₆alkylene-C₁₋₆alkoxy, or a C₃₋₈cycloalkyl;
R₆ is each independently chosen from a halogen, a C₁₋₆alkyl, a C₁₋₆haloalkyl, a C₁₋₆alkoxy, a C₁₋₆haloalkoxy, a C₃₋₈cycloalkyl, a 3-7 membered heterocyclyl, a 5-6 membered heteroaryl, or a phenyl;
each R₇ is independently chosen from hydrogen, a C₁₋₆alkyl, or a -(C₁₋₆alkylene)-OH, or the two R₇ are taken together with the nitrogen atom attached to the two R₇ to form a heterocycle optionally comprising one or more substituents each independently chosen from a halogen or an - OH substituent; and
n is 0, 1, 2, or 3.

In some embodiments, the compound of Formula I is a compound of Formula I-a: 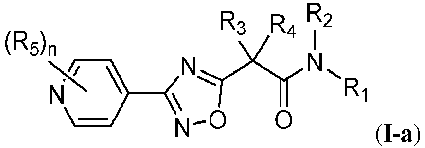 or a pharmaceutically acceptable salt thereof.

In some embodiments, the compound of Formula I is a compound of Formula **I-a1**: 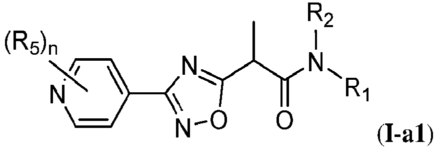 or a pharmaceutically acceptable salt thereof.

In some embodiments, the compound of Formula I is a compound of Formula **I-a2** or Formula **I-a3:** 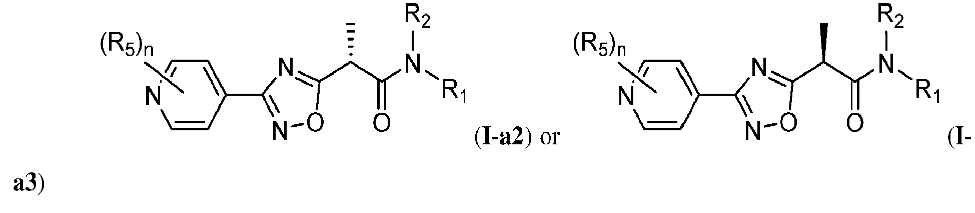 or a pharmaceutically acceptable salt thereof.

In some embodiments, the compound of Formula I is a compound of Formula **I-a4** or Formula **I-a5:** 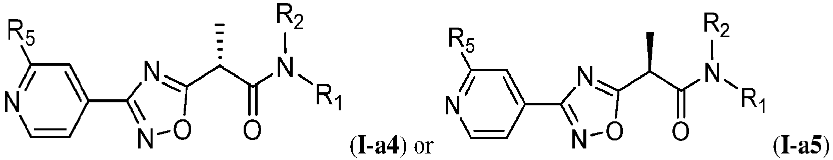 or a pharmaceutically acceptable salt thereof.

In certain embodiments of the compound of Formula (I) or a pharmaceutically acceptable salt thereof, R₁ is chosen from a C₁₋₆alkyl, a C₃₋₈cycloalkyl, or a phenyl, each optionally comprising one R₆ substituent, and in certain embodiments, R₁ is a C₁₋₆alkyl comprising one R₆ substituent. In certain embodiments, R₁ is a C₁₋₆alkyl comprising a phenyl substituent; in certain embodiments, R₁ is a C₃₋₈cycloalkyl, and in certain embodiments, R₁ is a cyclohexyl. In certain embodiments, R₁ is a phenyl, and in certain embodiments, R₁ and R₂ are taken together with the nitrogen attached to R₁ and R₂ to form a 3-7 membered heterocyclyl. In certain embodiments disclosed herein, R₂ is hydrogen; in certain embodiments, R₃ is a C₁₋₆alkyl, and in certain embodiments, R₃ is a methyl. In certain embodiments, R₄ is a hydrogen; in certain embodiments, n is 1, and in certain embodiments, R₅ is a C₃₋₈cycloalkyl.

In certain embodiments, the compound of Formula (I) is chosen from: 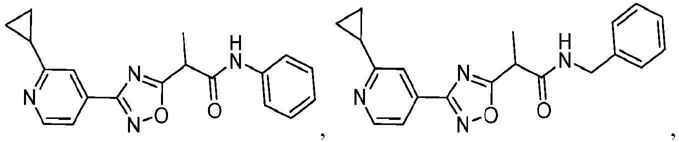 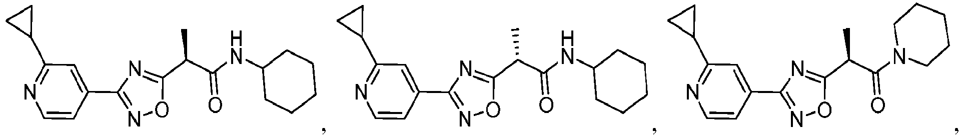 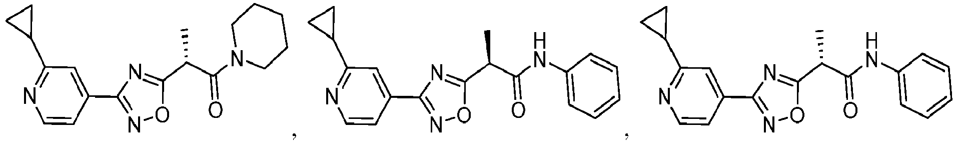 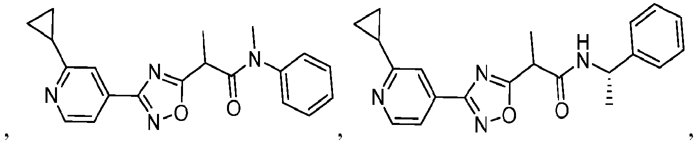 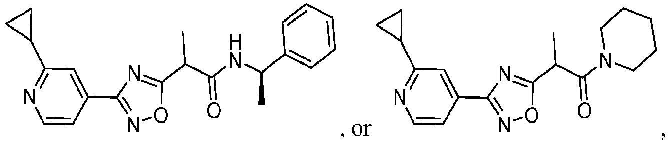 or a pharmaceutically acceptable salt thereof.

Also disclosed herein are pharmaceutical compositions comprising a compound or pharmaceutically acceptable salt thereof as disclosed herein, and a pharmaceutically acceptable excipient.

In one aspect, the present disclosure provides a method of treating neurological disorder, wherein the method comprises administering to a subject in need thereof a compound disclosed herein (e.g., compound of Formula (I) (e.g., (I-a), (I-a1), (I-a2), (I-a3), (I-a4), or (I-a5))) or a pharmaceutically acceptable salt thereof or a pharmaceutical composition disclosed herein (e.g., a pharmaceutical composition comprising a compound of Formula (I) (e.g., (I-a), (I-a1), (I-a2), (I-a3), (I-a4), or (I-a5)), or a pharmaceutically acceptable salt thereof, and a pharmaceutically acceptable excipient).

In another aspect, the present disclosure provides a method of treating a disorder associated with excessive neuronal excitability, wherein the method comprises administering to a subject in need thereof a compound disclosed herein (e.g., compound of Formula (I) (e.g., (I-a), (I-a1), (I-a2), (I-a3), (I-a4), or (I-a5))) or a pharmaceutically acceptable salt thereof or a pharmaceutical composition disclosed herein (e.g., a pharmaceutical composition comprising a compound of Formula (I) (e.g., (I-a), (I-a1), (I-a2), (I-a3), (I-a4), or (I-a5)), or a pharmaceutically acceptable salt thereof, and a pharmaceutically acceptable excipient).

In another aspect, the present disclosure provides a method of treating a disorder associated with a gain-of-function mutation of a gene (e.g., KCNT1), wherein the method comprises administering to a subject in need thereof a compound disclosed herein (e.g., a compound of Formula (I) (e.g., (I-a), (I-a1), (I-a2), (I-a3), (I-a4), or (I-a5))) or a pharmaceutically acceptable salt thereof or a pharmaceutical composition disclosed herein (e.g., a pharmaceutical composition comprising a compound of Formula (I) (e.g., (I-a), (I-a1), (I-a2), (I-a3), (I-a4), or (I-a5))), or a pharmaceutically acceptable salt thereof, and a pharmaceutically acceptable excipient).

In some embodiments, the neurological disorder, the disorder associated with excessive neuronal excitability, or the disorder associated with a gain-of-function mutation of a gene (e.g., KCNT1) is epilepsy, an epilepsy syndrome, or an encephalopathy.

In some embodiments, the neurological disorder, the disorder associated with excessive neuronal excitability, or the disorder associated with a gain-of-function mutation of a gene (e.g., KCNT1) is a genetic or pediatric epilepsy or a genetic or pediatric epilepsy syndrome.

In some embodiments, the neurological disorder, the disorder associated with excessive neuronal excitability, or the disorder associated with a gain-of-function mutation of a gene (e.g., KCNT1) is a cardiac dysfunction.

In some embodiments, the neurological disorder, the disorder associated with excessive neuronal excitability, or the disorder associated with a gain-of-function mutation of a gene (e.g., KCNT1) is selected from epilepsy and other encephalopathies (e.g., malignant migrating focal seizures of infancy (MMFSI) or epilepsy of infancy with migrating focal seizures (EIMFS), autosomal dominant nocturnal frontal lobe epilepsy (ADNFLE), West syndrome, infantile spasms, epileptic encephalopathy, focal epilepsy, Ohtahara syndrome, developmental and epileptic encephalopathy, Lennox-Gastaut syndrome, seizures (e.g., Generalized tonic clonic seizures, Asymmetric Tonic Seizures), leukodystrophy, leukoencephalopathy, intellectual disability, Multifocal Epilepsy, Drug resistant epilepsy, Temporal lobe epilepsy, cerebellar ataxia).

In some embodiments, the neurological disorder, the disorder associated with excessive neuronal excitability, or the disorder associated with a gain-of-function mutation of a gene (e.g., KCNT1) is chosen from cardiac arrhythmia, sudden unexpected death in epilepsy, Brugada syndrome, or myocardial infarction.

In some embodiments, the neurological disorder, the disorder associated with excessive neuronal excitability, or the disorder associated with a gain-of-function mutation of a gene (e.g., KCNT1) is selected from pain and related conditions (e.g. neuropathic pain, acute/chronic pain, migraine, etc).

In some embodiments, the neurological disorder, the disorder associated with excessive neuronal excitability, or the disorder associated with a gain-of-function mutation of a gene (e.g., KCNT1) is a muscle disorder (e.g., myotonia, neuromyotonia, cramp muscle spasms, spasticity).

In some embodiments, the neurological disorder, the disorder associated with excessive neuronal excitability, or the disorder associated with a gain-of-function mutation of a gene (e.g., KCNT1) is selected from itch and pruritis, ataxia, or cerebellar ataxias.

In some embodiments, the neurological disorder, the disorder associated with excessive neuronal excitability, or the disorder associated with a gain-of-function mutation of a gene (e.g., KCNT1) is selected from psychiatric disorders (e.g., major depression, anxiety, bipolar disorder, schizophrenia).

In some embodiments, the neurological disorder, the disorder associated with excessive neuronal excitability, or the disorder associated with a gain-of-function mutation in a gene (e.g., KCNT1) is chosen from a learning disorder, Fragile X, neuronal plasticity, or an autism spectrum disorder.

In some embodiments, the neurological disorder, the disorder associated with excessive neuronal excitability, or the disorder associated with a gain-of-function mutation of a gene (e.g., KCNT1) is chosen from epileptic encephalopathy with SCN1A, SCN2A, and/or SCN8A mutations, early infantile epileptic encephalopathy, Dravet syndrome, Dravet syndrome with SCN1A mutation, generalized epilepsy with febrile seizures, intractable childhood epilepsy with generalized tonic-clonic seizures, infantile spasms, benign familial neonatal-infantile seizures, SCN2A epileptic encephalopathy, focal epilepsy with SCN3A mutation, cryptogenic pediatric partial epilepsy with SCN3A mutation, SCN8A epileptic encephalopathy, sudden unexpected death in epilepsy, Rasmussen encephalitis, malignant migrating partial seizures of infancy, autosomal dominant nocturnal frontal lobe epilepsy, sudden expected death in epilepsy (SUDEP), KCNQ2 epileptic encephalopathy, or KCNT1 epileptic encephalopathy.

Other objects and advantages will become apparent to those skilled in the art from a consideration of the ensuing **Detailed Description, Examples,** and **Claims.**

### DETAILED DESCRIPTION OF THE DISCLOSURE

Provided herein are compounds and compositions useful for preventing and/or treating a disease, disorder, or condition described herein, e.g., a neurological disorder, a disorder associated with excessive neuronal excitability, or a disorder associated with gain-of-function mutations in a gene (e.g., KCNT1). Exemplary diseases, disorders, or conditions include epilepsy and other encephalopathies (e.g., MMFSI or EIMFS, ADNFLE, West syndrome, infantile spasms, epileptic encephalopathy, focal epilepsy, Ohtahara syndrome, developmental and epileptic encephalopathy, and Lennox-Gastaut syndrome, seizures, leukodystrophy, leukoencephalopathy, Intellectual disability, Multifocal Epilepsy, Generalized tonic clonic seizures, Drug resistant epilepsy, Temporal lobe epilepsy, cerebellar ataxia, Asymmetric Tonic Seizures); cardiac dysfunctions (e.g., cardiac arrhythmia, Brugada syndrome, myocardial infarction); pain and related conditions (e.g., neuropathic pain, acute/chronic pain, migraine, etc); muscle disorders (e.g., myotonia, neuromyotonia, cramp muscle spasms, spasticity); itch and pruritis; ataxia and cerebellar ataxias; and psychiatric disorders (e.g., major depression, anxiety, bipolar disorder, schizophrenia).

### Definitions

### Chemical definitions

Definitions of specific functional groups and chemical terms are described in more detail below. The chemical elements are identified in accordance with the Periodic Table of the Elements, CAS version, *Handbook of Chemistry and Physics,* 75^{th} Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods described in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The compounds described herein encompass both individual isomers substantially free of other isomers, and alternatively, mixtures of various isomers.

As used herein, a pure enantiomeric compound is substantially free from other enantiomers or stereoisomers of the compound (*i.e.,* in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the enantiomer. In certain embodiments, the weights are based upon total weight of all enantiomers or stereoisomers of the compound.

In the compositions provided herein, an enantiomerically pure compound can be present with other active or inactive ingredients. For example, a pharmaceutical composition comprising enantiomerically pure R-compound can comprise, for example, about 90% excipient and about 10% enantiomerically pure R-compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of the compound. In certain embodiments, the active ingredient can be formulated with little or no excipient or carrier.

Compound described herein may also comprise one or more isotopic substitutions. For example, H may be in any isotopic form, including ¹H, ²H (D or deuterium), and ³H (T or tritium); C may be in any isotopic form, including ¹²C, ¹³C, and ¹⁴C; O may be in any isotopic form, including ¹⁶O and ¹⁸O; F may be in any isotopic form, including ¹⁸F and ¹⁹F; and the like.

The following terms are intended to have the meanings presented therewith below and are useful in understanding the description and intended scope of the present disclosure. When describing the embodiments, which may include compounds and pharmaceutically acceptable salts thereof, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein. The articles "a" and "an" may be used herein to refer to one or to more than one (*i.e.* at least one) of the grammatical objects of the article. By way of example "an analogue" means one analogue or more than one analogue.

When a range of values is listed, it is intended to encompass each value and sub-range within the range. For example, "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

As used herein, "alkyl" refers to a radical of a straight-chain or branched saturated hydrocarbon group, e.g., having 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). Examples of C₁₋₆ alkyl groups include methyl, ethyl, propyl, isopropyl, butyl, isobutyl, pentyl, hexyl, and the like.

The term "heteroalkyl" as used herein refers to an "alkyl" group in which at least one carbon atom has been replaced with an O or S atom. The heteroalkyl may be, for example, an -O-C₁-C₁₀alkyl group, an -C₁-C₆alkylene-O-C₁-C₆alkyl group, or a C₁-C₆ alkylene-OH group. In certain embodiments, the "heteroalkyl" may be 2-8 membered heteroalkyl, indicating that the heteroalkyl contains from 2 to 8 atoms selected from the group consisting of carbon, oxygen, nitrogen, and sulfur. In yet other embodiments, the heteroalkyl may be a 2-6 membered, 4-8 membered, or a 5-8 membered heteroalkyl group (which may contain for example 1 or 2 heteroatoms selected from the group oxygen and nitrogen). In certain embodiments, the heteroalkyl is an "alkyl" group in which 1-3 carbon atoms have been replaced with oxygen atoms. One type of heteroalkyl group is an "alkoxy" group.

As used herein, "alkenyl" refers to a radical of a straight-chain or branched hydrocarbon group having from 2 to 20 carbon atoms, one or more carbon-carbon double bonds (e.g., 1, 2, 3, or 4 carbon-carbon double bonds), and optionally one or more carbon-carbon triple bonds (e.g., 1, 2, 3, or 4 carbon-carbon triple bonds) ("C₂₋₂₀ alkenyl"). In certain embodiments, alkenyl does not contain any triple bonds. In some embodiments, an alkenyl group has 2 to 10 carbon atoms ("C₂₋₁₀ alkenyl"). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅). hexenyl (C₆), and the like. Additional examples of alkenyl include heptenyl (C₇), octenyl (C₈), octatrienyl (C₈), and the like.

As used herein, "alkynyl" refers to a radical of a straight-chain or branched hydrocarbon group having from 2 to 20 carbon atoms, one or more carbon-carbon triple bonds (e.g., 1, 2, 3, or 4 carbon-carbon triple bonds), and optionally one or more carbon-carbon double bonds (e.g., 1, 2, 3, or 4 carbon-carbon double bonds) ("C₂₋₂₀ alkynyl"). In certain embodiments, alkynyl does not contain any double bonds. In some embodiments, an alkynyl group has 2 to 10 carbon atoms ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like.

As used herein, "alkylene," "alkenylene," and "alkynylene," refer to a divalent radical of an alkyl, alkenyl, and alkynyl group respectively. When a range or number of carbons is provided for a particular "alkylene," "alkenylene," or "alkynylene," group, it is understood that the range or number refers to the range or number of carbons in the linear carbon divalent chain. "Alkylene," "alkenylene," and "alkynylene," groups may be substituted or unsubstituted with one or more substituents as described herein.

As used herein, "aryl" refers to a radical of a monocyclic or polycyclic (e.g., bicyclic or tricyclic) 4n+2 aromatic ring system (e.g., having 6, 10, or 14 π electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, an aryl group has fourteen ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl.

As used herein, "heteroaryl" refers to a radical of a 5-10 membered monocyclic or bicyclic 4n+2 aromatic ring system (e.g., having 6 or 10 electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (e.g., indolyl, quinolinyl, carbazolyl, and the like) the point of attachment can be on either ring, i.e., either the ring bearing a heteroatom (e.g., 2-indolyl) or the ring that does not contain a heteroatom (e.g., 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Examples of representative heteroaryls include the following: 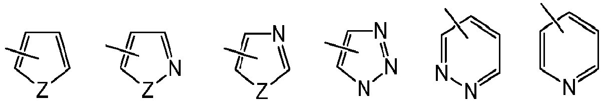 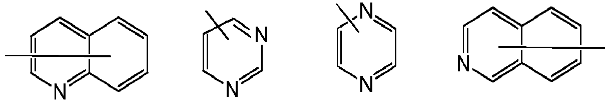 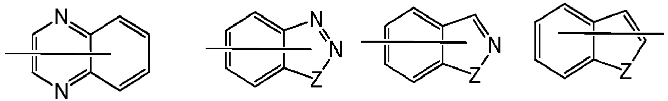 wherein each Z is selected from carbonyl, N, NR⁶⁵, O, and S; and R⁶⁵ is independently hydrogen, C₁-C₈ alkyl, C₃-C₁₀ carbocyclyl, 4-10 membered heterocyclyl, C₆-C₁₀ aryl, and 5-10 membered heteroaryl.

As used herein, "carbocyclyl" or "carbocyclic" refers to a radical of a non-aromatic cyclic hydrocarbon group having from 3 to 10 ring carbon atoms ("C₃₋₁₀ carbocyclyl") and zero heteroatoms in the non-aromatic ring system. In some embodiments, a carbocyclyl group has 3 to 8 ring carbon atoms ("C₃₋₈ carbocyclyl"). In some embodiments, a carbocyclyl group has 3 to 6 ring carbon atoms ("C₃₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃),cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1H-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system.

The term "cycloalkyl" refers to a monovalent saturated cyclic, bicyclic, or bridged cyclic (e.g., adamantyl) hydrocarbon group of 3-12, 3-8, 4-8, or 4-6 carbons, referred to herein, e.g., as "C₄₋₈cycloalkyl," derived from a cycloalkane. Exemplary cycloalkyl groups include, but are not limited to, cyclohexanes, cyclopentanes, cyclobutanes and cyclopropanes. Unless specified otherwise, cycloalkyl groups are optionally substituted at one or more ring positions with, for example, alkanoyl, alkoxy, alkyl, haloalkyl, alkenyl, alkynyl, amido, amidino, amino, aryl, arylalkyl, azido, carbamate, carbonate, carboxy, cyano, cycloalkyl, ester, ether, formyl, halogen, haloalkyl, heteroaryl, heterocyclyl, hydroxyl, imino, ketone, nitro, phosphate, phosphonato, phosphinato, sulfate, sulfide, sulfonamido, sulfonyl or thiocarbonyl. Cycloalkyl groups can be fused to other cycloalkyl, aryl, or heterocyclyl groups. In certain embodiments, the cycloalkyl group is not substituted, i.e., it is unsubstituted.

As used herein, "heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, e.g., heteroalkyl; carbocyclyl, e.g., heterocyclyl; aryl, e.g., heteroaryl; and the like having from 1 to 5, and particularly from 1 to 3 heteroatoms.

As used herein, "cyano" refers to -CN.

As used herein, "halo" or "halogen" refers to fluoro (F), chloro (Cl), bromo (Br) and iodo (I). In certain embodiments, the halo group is either fluoro or chloro.

As used herein, "haloalkyl" refers to an alkyl group substituted with one or more halogen atoms.

As used herein, "nitro" refers to -NO₂.

In general, the term "substituted", whether preceded by the term "optionally" or not, means that at least one hydrogen present on a group (e.g., a carbon or nitrogen atom) is replaced with a permissible substituent, e.g., a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position.

Nitrogen atoms can be substituted or unsubstituted as valency permits, and include primary, secondary, tertiary, and quarternary nitrogen atoms. Exemplary nitrogen atom substituents include, but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, - C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, C₁₋₁₀ alkyl, C₁₋₁₀ perhaloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{cc} groups attached to a nitrogen atom are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc} and R^{dd} are as defined above.

These and other exemplary substituents are described in more detail in the **Detailed Description,** Examples, and Claims. The disclosure is not intended to be limited in any manner by the above exemplary listing of substituents.

### Other definitions

The terms "in some embodiments," "in other embodiments," or the like, refer to embodiments of all aspects of the disclosure, unless the context clearly indicated otherwise.

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. The general concept of pharmaceutically acceptable salts has been discussed in the art, including, for example, Berge et al., which describes pharmaceutically acceptable salts in detail in J Pharmaceutical Sciences (1977) 66: 1-19. Pharmaceutically acceptable salts of the compounds described herein include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

As used herein, a "subject" to which administration is contemplated includes, but is not limited to, humans (i.e., a male or female of any age group, e.g., a pediatric subject (e.g., an infant, child, adolescent) or an adult subject (e.g., a young adult, middle-aged adult, or senior adult)) and/or a non-human animal, e.g., a mammal such as primates (e.g., cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human," "patient," and "subject" are used interchangeably herein.

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition (also "therapeutic treatment").

In general, the "effective amount" of a compound or pharmaceutically acceptable salt thereof refers to an amount sufficient to elicit the desired biological response. As will be appreciated by those of ordinary skill in this art, the effective amount of a compound or pharmaceutically acceptable salt thereof as disclosed herein may vary depending on such factors as the desired biological endpoint, the pharmacokinetics of the compound or pharmaceutically acceptable salt thereof, the disease being treated, the mode of administration, and the age, weight, health, and condition of the subject.

As used herein, and unless otherwise specified, a "therapeutically effective amount" of a compound or pharmaceutically acceptable salt thereof is an amount sufficient to provide a therapeutic benefit in the treatment of a disease, disorder or condition, or to delay or minimize one or more symptoms associated with the disease, disorder or condition. A therapeutically effective amount of a compound or pharmaceutically acceptable salt thereof means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

In certain embodiments, provided herein are methods of treating comprising administering the compounds disclosed herein or a pharmaceutically acceptable salt or a pharmaceutically acceptable composition thereof, as a prophylactic before a subject begins to suffer from the specified disease, disorder or condition. As used herein, "prophylactic treatment" contemplates an action that occurs before a subject begins to suffer from the specified disease, disorder or condition. As used herein, and unless otherwise specified, a "prophylactically effective amount" of a compound or pharmaceutically acceptable salt thereof is an amount sufficient to prevent a disease, disorder or condition, or one or more symptoms associated with the disease, disorder or condition, or prevent its recurrence. A prophylactically effective amount of a compound or pharmaceutically acceptable salt thereof means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

As used herein, a "disorder associated with a gain-of-function mutation in KCNT1" refers to a disorder that is associated with, is partially or completely caused by, or has one or more symptoms that are partially or completely caused by, a mutation in KCNT1 that results in a gain-of-function phenotype, i.e., an increase in activity of the potassium channel encoded by KCNT1 resulting in an increase in whole cell current.

As used herein, a "gain-of-function mutation" is a mutation in KCNT1 that results in an increase in activity of the potassium channel encoded by KCNT1. Activity can be assessed by, for example, ion flux assay or electrophysiology (e.g., using the whole cell patch clamp technique). Typically, a gain-of-function mutation results in an increase of at least or about 20%, 30%, 40%, 50%, 60%, 70%, 80%, 90%, 100%, 125%, 150%, 175%, 200%, 225%, 250%, 275%, 300%, 325%, 350%, 375%, 400% or more compared to the activity of a potassium channel encoded by a wild-type KCNT1.

### Compounds and Compositions

In one aspect, disclosed herein is a compound of Formula (I) having an oxadiazole core: 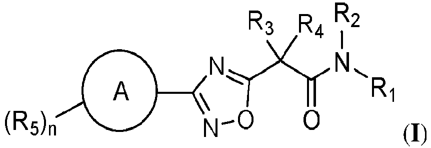 or a pharmaceutically acceptable salt thereof, wherein
ring A is a 5-6 membered heteroaryl;
R₁ is chosen from a C₁₋₆alkyl, a C₃₋₈cycloalkyl, a phenyl, a 3-7 membered heterocyclyl, or a 5-6 membered heteroaryl, wherein the C₁₋₆alkyl, C₃₋₈cycloalkyl, phenyl, 3-7 membered heterocyclyl, or 5-6 membered heteroaryl optionally comprises one or more R₆ substituents;
R₂ is chosen from hydrogen or a C₁₋₆alkyl; or
R₁ and R₂ are taken together with the nitrogen attached to R₁ and R₂ to form a 3-7 membered heterocyclyl optionally comprising one or more R₆ substituents;
R₃ is chosen from a hydrogen or a C₁₋₆alkyl optionally comprising one or more substituents chosen from a halogen, a C₁₋₆haloalkyl, a C₁₋₆alkoxy, or a C₁₋₆haloalkoxy;
R₄ is a C₁₋₆alkyl optionally comprising one or more substituents chosen from a halogen, a C₁₋₆haloalkyl, a C₁₋₆alkoxy, or a C₁₋₆haloalkoxy; or
R₃ and R₄ are taken together with the carbon attached to R₃ and R₄ to form a C₃₋₈cycloalkylene or a 3-7 membered heterocycloalkylene;
R₅ is each independently chosen from a halogen, a C₁₋₆alkyl, a C₁₋₆haloalkyl, a C₁₋₆alkoxy, a C₁₋₆haloalkoxy, -N(R₇)₂, a C₁₋₆alkylene-C₁₋₆alkoxy, or a C₃₋₈cycloalkyl;
R₆ is each independently chosen from a halogen, a C₁₋₆alkyl, a C₁₋₆haloalkyl, a C₁₋₆alkoxy, a C₁₋₆haloalkoxy, a C₃₋₈cycloalkyl, a 3-7 membered heterocyclyl, a 5-6 membered heteroaryl, or a phenyl;
each R₇ is independently chosen from hydrogen, a C₁₋₆alkyl, or a -(C₁₋₆alkylene)-OH, or the two R₇ are taken together with the nitrogen atom attached to the two R₇ to form a heterocycle optionally comprising one or more substituents each independently selected from halogen or - OH; and
n is 0, 1, 2, or 3.

In some embodiments, the compound of Formula I is a compound of Formula **I-a:** 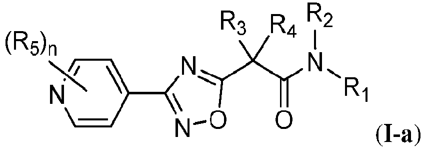 or a pharmaceutically acceptable salt thereof.

In some embodiments, the compound of Formula I is a compound of Formula **I-a1:** 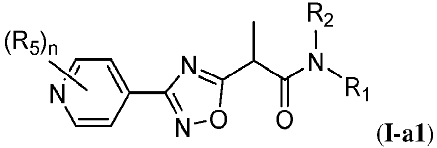 or a pharmaceutically acceptable salt thereof.

In some embodiments, the compound of Formula I is a compound of Formula **I-a2** or Formula **I-a3:** 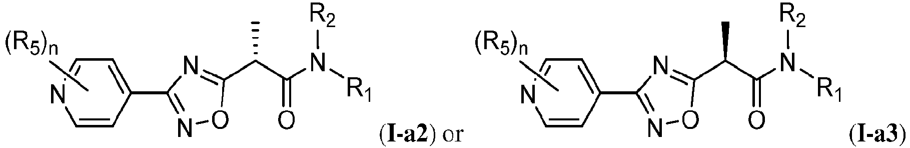 or a pharmaceutically acceptable salt thereof.

In some embodiments, the compound of Formula I is a compound of Formula **I-a4** or Formula **I-a5:** 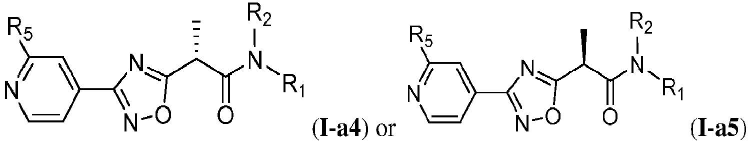 or a pharmaceutically acceptable salt thereof.

In some embodiments of Formula I, I-a, I-a1, I-a2, I-a3, I-a4, or I-a5, R₁ is chosen from a C₁₋₆alkyl, a C₃₋₈cycloalkyl, or a phenyl, each optionally comprising one R₆ substituent.

In some embodiments of Formula I, I-a, I-a1, I-a2, I-a3, I-a4, or I-a5, R₁ is a C₁₋₆alkyl comprising one R₆ (e.g., phenyl) substituent.

In some embodiments of Formula I, I-a, I-a1, I-a2, I-a3, I-a4, or I-a5, R₁ is a C₃₋₈cycloalkyl (e.g., cyclohexyl).

In some embodiments of Formula I, I-a, I-a1, I-a2, I-a3, I-a4, or I-a5, R₁ is a phenyl.

In some embodiments of Formula I, I-a, I-a1, I-a2, I-a3, I-a4, or I-a5, R₁ and R₂ are taken together with the nitrogen attached to R₁ and R₂ to form a 3-7 membered heterocyclyl.

In some embodiments of Formula I, I-a, I-a1, I-a2, I-a3, I-a4, or I-a5, R₂ is hydrogen.

In some embodiments of Formula I, I-a, or I-a1, R₃ is a C₁₋₆alkyl (e.g., methyl).

In some embodiments of Formula I, I-a, or I-a1, R₄ is hydrogen.

In some embodiments of Formula I, I-a, I-a1, I-a2, or I-a3, n is 1.

In some embodiments of Formula I, I-a, I-a1, I-a2, I-a3, I-a4, or I-a5, R₅ is a C₃₋₈cycloalkyl (e.g., cyclopropyl).

In some embodiments, the compound is chosen from : 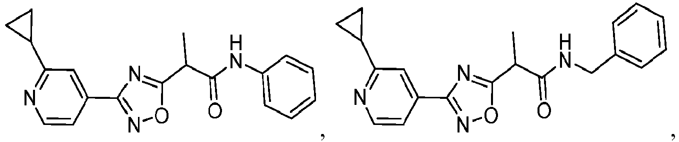 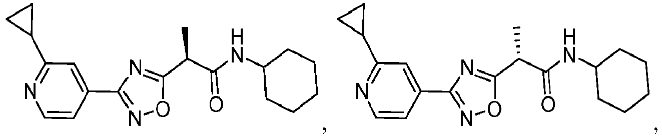 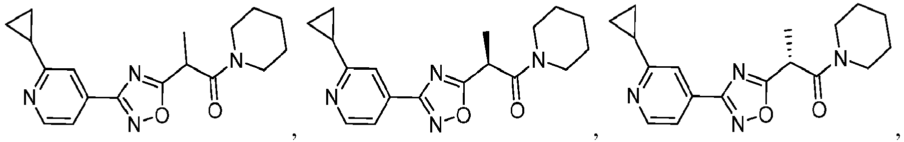 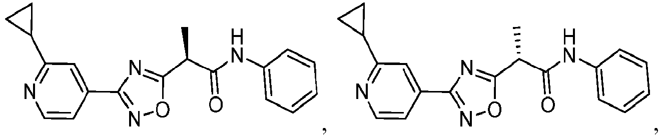 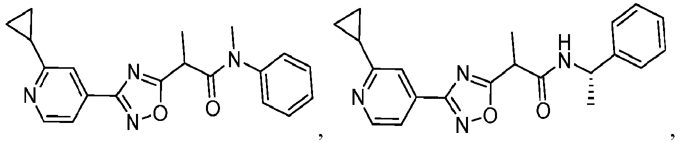 or 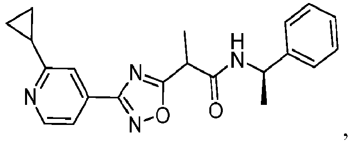 or a pharmaceutically acceptable salt thereof.

In another aspect, provided herein is a pharmaceutical composition comprising a compound disclosed herein or a pharmaceutically acceptable salt thereof and a pharmaceutically acceptable excipient.

### General Synthetic Schemes

Exemplary methods for preparing compounds described herein are illustrated in the following synthetic schemes. These schemes are given for the purpose of illustrating exemplary embodiments of the disclosure, and should not be regarded in any manner as limiting the scope or the spirit of the disclosure.

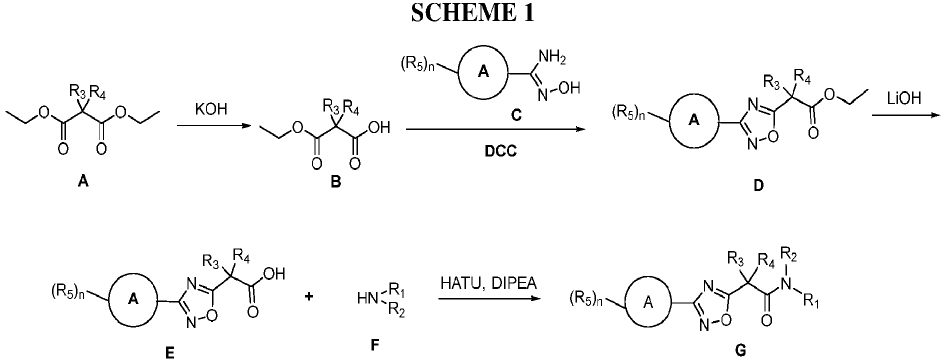

The synthetic route illustrated in Scheme 1 depicts an exemplary procedure for preparing a compound of Formula I. In the first step, malonate **A** is treated with potassium hydroxide to provide carboxylic acid **B,** which is then treated with *N*-hydroxyimidamide **C** and *N,N'*-dicyclohexylcarbodiimide (DCC) to afford oxadiazole **D.** Hydrolysis of **D** under basic conditions provides intermediate **E,** which is then treated with amine **F** under standard peptide coupling procedures (e.g., HATU in dichloromethane in the presence of DIPEA) to provide compound **G** (a compound of Formula I).

### Methods of Treatment

The compounds and compositions described above and herein can be used to treat a neurological disorder, a disorder associated with excessive neuronal excitability, or disorder associated with a gain-of-function mutation in a gene (e.g., KCNT1). Exemplary diseases, disorders, or conditions include epilepsy and other encephalopathies (e.g., MMFSI or EIMFS, ADNFLE, West syndrome, infantile spasms, epileptic encephalopathy, developmental and epileptic encephalopathy (DEE), early infantile epileptic encephalopathy (EIEE), generalized epilepsy, focal epilepsy, multifocal epilepsy, temporal lobe epilepsy, Ohtahara syndrome, early myoclonic encephalopathy, Lennox-Gastaut syndrome, drug-resistant epilepsy, seizures (e.g., frontal lobe seizures, generalized tonic clonic seizures, asymmetric tonic seizures, focal seizures), leukodystrophy, hypomyelinating leukodystrophy, and leukoencephalopathy), cardiac dysfunctions (e.g., cardiac arrhythmia, Brugada syndrome, myocardial infarction), pulmonary vasculopathy / hemorrhage, pain and related conditions (e.g., neuropathic pain, acute/chronic pain, migraine, etc.), muscle disorders (e.g., myotonia, neuromyotonia, cramp muscle spasms, spasticity), itch and pruritis, movement disorders (e.g., ataxia and cerebellar ataxias), psychiatric disorders (e.g., major depression, anxiety, bipolar disorder, schizophrenia, attention-deficit hyperactivity disorder), neurodevelopmental disorder, learning disorders, intellectual disability, Fragile X, neuronal plasticity, and autism spectrum disorders.

In some embodiments, the neurological disorder, the disorder associated with excessive neuronal excitability, or the disorder associated with a gain-of-function mutation in a gene (e.g., KCNT1) is selected from EIMFS, ADNFLE and West syndrome. In some embodiments, the neurological disorder, the disorder associated with excessive neuronal excitability, or the disorder associated with a gain-of-function mutation in a gene (e.g., KCNT1) is selected from infantile spasms, epileptic encephalopathy, focal epilepsy, Ohtahara syndrome, developmental and epileptic encephalopathy and Lennox-Gastaut syndrome. In some embodiments, the neurological disorder, the disorder associated with excessive neuronal excitability, or the disorder associated with a gain-of-function mutation in a gene (e.g., KCNT1) is seizure. In some embodiments, the neurological disorder, the disorder associated with excessive neuronal excitability, or the disorder associated with a gain-of-function mutation in a gene (e.g., KCNT1) is selected from cardiac arrhythmia, Brugada syndrome, and myocardial infarction.

In some embodiments, the neurological disorder, the disorder associated with excessive neuronal excitability, or the disorder associated with a gain-of-function mutation in a gene (e.g., KCNT1) is selected from a learning disorder, Fragile X, intellectual function, neuronal plasticity, a psychiatric disorder, or an autism spectrum disorder.

Accordingly, the compounds and compositions thereof can be administered to a subject with a neurological disorder, a disorder associated with excessive neuronal excitability, or a disorder associated with a gain-of-function mutation in a gene such as KCNT1 (e.g., EIMFS, ADNFLE, West syndrome, infantile spasms, epileptic encephalopathy, focal epilepsy, Ohtahara syndrome, developmental and epileptic encephalopathy, Lennox-Gastaut syndrome, seizures, cardiac arrhythmia, Brugada syndrome, and myocardial infarction).

EIMFS is a rare and debilitating genetic condition characterized by an early onset (before 6 months of age) of almost continuous heterogeneous focal seizures, where seizures appear to migrate from one brain region and hemisphere to another. Patients with EIMFS are generally intellectually impaired, non-verbal and non-ambulatory. While several genes have been implicated to date, the gene that is most commonly associated with EIMFS is KCNT1. Several de novo mutations in KCNT1 have been identified in patients with EIMFS, including V271F, G288S, R428Q, R474Q, R474H, R474C, I760M, A934T, P924L, G243S, H257D, A259D, R262Q, Q270E, L274I, F346L, C377S, R398Q, P409S, A477T, F502V, M516V, Q550del, K629E, K629N, I760F, E893K, M896K, R933G, R950Q, K1154Q. Barcia et al. (2012) Nat Genet. 44: 1255-1260; Ishii et al. (2013) Gene 531:467-471; McTague et al. (2013) Brain. 136: 1578-1591; Epi4K Consortium & Epilepsy Phenome/Genome Project. (2013) Nature 501:217-221; Lim et al. (2016) Neurogenetics; Ohba et al. (2015) Epilepsia 56:e121-e128; Zhou et al. (2018) Genes Brain Behav. e12456; Moller et al. (2015) Epilepsia. e114-20; Numis et al. (2018) Epilepsia. 1889-1898; Madaan et al. Brain Dev. 40(3):229-232; McTague et al. (2018) Neurology. 90(1):e55-e66; Kawasaki et al. (2017) J Pediatr. 191:270-274; Kim et al. (2014) Cell Rep. 9(5):1661-1672; Ohba et al. (2015) Epilepsia. 56(9):e121-8; Rizzo et al. (2016) Mol Cell Neurosci. 72:54-63; Zhang et al. (2017) Clin Genet. 91(5):717-724; Mikati et al. (2015) Ann Neurol. 78(6):995-9; Baumer et al. (2017) Neurology. 89(21):2212; Dilena et al. (2018) Neurotherapeutics. 15(4):1112-1126. These mutations may be gain-of-function, missense mutations that are dominant (i.e., present on only one allele) and result in change-in-function of the encoded potassium channel that causes a marked increase in whole cell current when tested in Xenopus oocyte or mammalian expression systems (see e.g. Milligan et al. (2015) Ann Neurol. 75(4): 581-590; Barcia et al. (2012) Nat Genet. 44(11): 1255-1259; and Mikati et al. (2015) Ann Neurol. 78(6): 995-999).

ADNFLE has a later onset than EIMFS, generally in mid-childhood, and is generally a less severe condition. It is characterized by nocturnal frontal lobe seizures and can result in psychiatric, behavioural and cognitive disabilities in patients with the condition. While ADNFLE is associated with genes encoding several neuronal nicotinic acetylcholine receptor subunits, mutations in the KCNT1 gene have been implicated in more severe cases of the disease (Heron et al. (2012) Nat Genet. 44: 1188-1190). Functional studies of the mutated KCNT1 genes associated with ADNFLE indicated that the underlying mutations (M896I, R398Q, Y796H and R928C) were dominant, gain-of-function mutations (Milligan et al. (2015) Ann Neurol. 75(4): 581-590; Mikati et al. (2015) Ann Neurol. 78(6): 995-999).

West syndrome is a severe form of epilepsy composed of a triad of infantile spasms, an interictal electroencephalogram (EEG) pattern termed hypsarrhythmia, and mental retardation, although a diagnosis can be made one of these elements is missing. Mutations in KCNT1, including G652V and R474H, have been associated with West syndrome (Fukuoka et al. (2017) Brain Dev 39:80-83 and Ohba et al. (2015) Epilepsia 56:e121-e128). Treatment targeting the KCNT1 channel suggests that these mutations are gain-of-function mutations (Fukuoka et al. (2017) Brain Dev 39:80-83).

In one aspect, disclosed herein is a method of treating a disorder associated with excessive neuronal excitability or disorder associated with a gain-of-function mutation in a gene such as KCNT1 (for example, epilepsy and other encephalopathies (e.g., MMFSI or EIMFS, ADNFLE, West syndrome, infantile spasms, epileptic encephalopathy, focal epilepsy, Ohtahara syndrome, DEE, Lennox-Gastaut syndrome, seizures, leukodystrophy, leukoencephalopathy, intellectual disability, Multifocal Epilepsy, Generalized tonic clonic seizures, drug-resistant epilepsy, Temporal lobe epilepsy, cerebellar ataxia, Asymmetric Tonic Seizures); cardiac dysfunctions (e.g., cardiac arrhythmia, Brugada syndrome, myocardial infarction); pain and related conditions (e.g., neuropathic pain, acute/chronic pain, migraine, etc.); muscle disorders (e.g., myotonia, neuromyotonia, cramp muscle spasms, spasticity); itch and pruritis; ataxia and cerebellar ataxias; psychiatric disorders (e.g., major depression, anxiety, bipolar disorder, schizophrenia); learning disorders, Fragile X, neuronal plasticity, and autism spectrum disorders) comprising administering to a subject in need thereof a compound disclosed herein (e.g., a compound of Formula (I) (e.g., (I-a1), (I-a2), (I-a3), (I-a4), or (I-a5))) or a pharmaceutically acceptable salt thereof) or a pharmaceutical composition disclosed herein (e.g., a pharmaceutical composition comprising a compound disclosed herein (e.g., a compound of Formula (I) (e.g., (I-a1), (I-a2), (I-a3), (I-a4), or (I-a5))) or a pharmaceutically acceptable salt thereof), and a pharmaceutically acceptable excipient).

In some examples, the subject presenting with a disorder that may be associated with a gain-of-function mutation in KCNT1 is genotyped to confirm the presence of a known gain-of-function mutation in KCNT1 prior to administration of the compounds or a pharmaceutically acceptable salt thereof or compositions discosed herein. For example, whole exome sequencing can be performed on the subject. Gain-of-function mutations associated with EIMFS may include, but are not limited to, V271F, G288S, R428Q, R474Q, R474H, R474C, I760M, A934T, P924L, G243S, H257D, A259D, R262Q, Q270E, L274I, F346L, C377S, R398Q, P409S, A477T, F502V, M516V, Q550del, K629E, K629N, I760F, E893K, M896K, R933G, R950Q, and K1154Q. Gain-of-function mutations associated with ADNFLE may include, but are not limited to, M896I, R398Q, Y796H, R928C, and G288S. Gain-of-function mutations associated with West syndrome may include, but are not limited to, G652V and R474H. Gain-of-function mutations associated with temporal lobe epilepsy may include, but are not limited to, R133H and R565H. Gain-of-function mutations associated with Lennox-Gastaut may include, but are not limited to, R209C. Gain-of-function mutations associated with seizures may include, but are not limited to, A259D, G288S, R474C, R474H. Gain-of-function mutations associated with leukodystrophy may include, but are not limited to, G288S and Q906H. Gain-of-function mutations associated with Multifocal Epilepsy may include, but are not limited to, V340M. Gain-of-function mutations associated with early-onset epilepsy (EOE) may include, but are not limited to, F346L and A934T. Gain-of-function mutations associated with Early-onset epileptic encephalopathies (EOEE) may include, but are not limited to, R428Q. Gain-of-function mutations associated with developmental and epileptic encephalopathies may include, but are not limited to, F346L, R474H, and A934T. Gain-of-function mutations associated with epileptic encephalopathies may include, but are not limited to, L437F, Y796H, P924L, and R961H. Gain-of-function mutations associated with Early Infantile Epileptic Encephalopathy (EIEE) may include, but are not limited to, M896K. Gain-of-function mutations associated with drug-resistant epilepsy and generalized tonic-clonic seizure may include, but are not limited to, F346L. Gain-of-function mutations associated with migrating partial seizures of infancy may include, but are not limited to, R428Q. Gain-of-function mutations associated with Leukoencephalopathy may include, but are not limited to, F932I. Gain-of-function mutations associated with NFLE may include, but are not limited to, A934T and R950Q. Gain-of-function mutations associated with Ohtahara syndrome may include, but are not limited to, A966T. Gain-of-function mutations associated with infantile spasms may include, but are not limited to, P924L. Gain-of-function mutations associated with Brugada Syndrome may include, but are not limited to, R1106Q. Gain-of-function mutations associated with Brugada Syndrome may include, but are not limited to, R474H.

In other examples, the subject is first genotyped to identify the presence of a mutation in KCNT1, and this mutation is then confirmed to be a gain-of-function mutation using standard in vitro assays, such as those described in Milligan et al. (2015) Ann Neurol. 75(4): 581-590. Typically, the presence of a gain-of-function mutation is confirmed when the expression of the mutated KCNT1 allele results an increase in whole cell current compared to the whole cell current resulting from expression of wild-type KCNT1, as may be assessed using whole-cell electrophysiology (such as described in Milligan et al. (2015) Ann Neurol. 75(4): 581-590; Barcia et al. (2012) Nat Genet. 44(11): 1255-1259; Mikati et al. (2015) Ann Neurol. 78(6): 995-999; or Rizzo et al. Mol Cell Neurosci. (2016) 72:54-63). This increase of whole cell current can be, for example, an increase of at least or about 50%, 100%, 150%, 200%, 250%, 300%, 350%, 400% or more. The subject can then be confirmed to have a disease or condition associated with a gain-of-function mutation in KCNT1.

In particular examples, the subject is confirmed as having a KCNT1 allele containing a gain-of-function mutation (e.g., V271F, G288S, R398Q, R428Q, R474Q, R474H, R474C, G652V, I760M, Y796H, M896I, P924L. R928C or A934T).

The compounds disclosed herein (e.g., a compound of Formula (I) (e.g., (I-a1), (I-a2), (I-a3), (I-a4), or (I-a5)) or a pharmaceutically acceptable salt thereof) or the pharmaceutical composition disclosed herein (e.g., a pharmaceutical composition comprising a compound disclosed herein (e.g., a compound of Formula (I) (e.g., (I-a1), (I-a2), (I-a3), (I-a4), or (I-a5)) or a pharmaceutically acceptable salt thereof), and a pharmaceutically acceptable excipient) can also be used therapeutically for conditions associated with excessive neuronal excitability where the excessive neuronal excitability is not necessarily the result of a gain-of-function mutation in KCNT1. Even in instances where the disease is not the result of increased KCNT1 expression and/or activity, inhibition of KCNT1 expression and/or activity can nonetheless result in a reduction in neuronal excitability, thereby providing a therapeutic effect. Thus, the compounds disclosed herein (e.g., a compound of Formula (I) (e.g., (I-a1), (I-a2), (I-a3), (I-a4), or (I-a5)) or a pharmaceutically acceptable salt thereof) or the pharmaceutical composition disclosed herein (e.g., a pharmaceutical composition comprising a compound disclosed herein (e.g., a compound of Formula (I) (e.g., (I-a1), (I-a2), (I-a3), (I-a4), or (I-a5)) or a pharmaceutically acceptable salt thereof), and a pharmaceutically acceptable excipient) can be used to treat a subject with conditions associated with excessive neuronal excitability, for example, epilepsy and other encephalopathies (e.g., EIMFS, ADNFLE, West syndrome, infantile spasms, epileptic encephalopathy, focal epilepsy, Ohtahara syndrome, developmental and epileptic encephalopathy, Lennox-Gastaut syndrome, seizures) or cardiac dysfunctions (e.g., cardiac arrhythmia, Brugada syndrome, myocardial infarction), regardless of whether or not the disorder is associated with a gain-of-function mutation in KCNT1.

### Pharmaceutical Compositions and Routes of Administration

Compounds disclosed herein and pharmaceutically acceptable salts thereof are usually administered in the form of pharmaceutical compositions. Therefore, disclosed herein are pharmaceutical compositions that contain, as the active ingredient, one or more of the compounds described, or a pharmaceutically acceptable salt or ester thereof, and one or more pharmaceutically acceptable excipients, carriers, including inert solid diluents and fillers, diluents, including sterile aqueous solution and various organic solvents, permeation enhancers, solubilizers and adjuvants. The pharmaceutical compositions may be administered alone or in combination with other therapeutic agents. Such compositions may be prepared in a manner disclosed in the pharmaceutical art, including, for example, in Remington's Pharmaceutical Sciences, Mace Publishing Co., Philadelphia, Pa. 17th Ed. (1985); and Modern Pharmaceutics, Marcel Dekker, Inc. 3rd Ed. (G. S. Banker & C. T. Rhodes, Eds.).

The pharmaceutical compositions may be administered in either single or multiple doses by any of the accepted modes of administration of agents having similar utilities, for example as described in those patents and patent applications incorporated by reference, including rectal, buccal, intranasal and transdermal routes, by intra-arterial injection, intravenously, intraperitoneally, parenterally, intramuscularly, subcutaneously, orally, topically, as an inhalant, or via an impregnated or coated device such as a stent, for example, or an artery-inserted cylindrical polymer.

One mode for administration is parenteral, particularly by injection. The forms in which the novel compositions disclosed herein may be incorporated for administration by injection include aqueous or oil suspensions, or emulsions, with sesame oil, corn oil, cottonseed oil, or peanut oil, as well as elixirs, mannitol, dextrose, or a sterile aqueous solution, and similar pharmaceutical vehicles. Aqueous solutions in saline are also conventionally used for injection. Ethanol, glycerol, propylene glycol, liquid polyethylene glycol, and the like (and suitable mixtures thereof), cyclodextrin derivatives, and vegetable oils may also be employed. The proper fluidity can be maintained, for example, by the use of a coating, such as lecithin, by the maintenance of the required particle size in the case of dispersions, and by the use of surfactants. The prevention of the action of microorganisms can be brought about by various antibacterial and antifungal agents, for example, parabens, chlorobutanol, phenol, sorbic acid, thimerosal, and the like.

Sterile injectable solutions are prepared by incorporating a compound or pharmaceutically acceptable salt thereof as disclosed herein in the desired amount in the appropriate solvent with various other ingredients as enumerated above, as desired, followed by filtered sterilization. Generally, dispersions are prepared by incorporating the various sterilized active ingredients into a sterile vehicle which contains the basic dispersion medium and the desired other ingredients from those enumerated above. In the case of sterile powders for the preparation of sterile injectable solutions, exemplary methods of preparation include vacuum-drying and freeze-drying techniques which yield a powder of the active ingredient plus any additional desired ingredient from a previously sterile-filtered solution thereof.

Oral administration is another route for administration of the compounds or pharmaceutically acceptable salt thereof as disclosed herein. Administration may be via capsule or enteric coated tablets, or the like. In making the pharmaceutical compositions that include at least one compound or pharmaceutically acceptable salts thereof described herein, the active ingredient may be diluted by an excipient and/or enclosed within such a carrier that can be in the form of a capsule, sachet, paper or other container. When the excipient serves as a diluent, it can be in the form of a solid, semi-solid, or liquid material (as above), which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, sterile injectable solutions, and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. In certain embodiments, the compositions disclosed herein can additionally include: lubricating agents such as talc, magnesium stearate, and mineral oil; wetting agents; emulsifying and suspending agents; preserving agents such as methyl and propylhydroxybenzoates; sweetening agents; and flavoring agents.

The compositions disclosed herein can be formulated so as to provide quick, sustained or delayed release of the active ingredient after administration to the patient. Controlled release drug delivery systems for oral administration include osmotic pump systems and dissolutional systems containing polymer-coated reservoirs or drug-polymer matrix formulations. Examples of controlled release systems are given in U.S. Pat. Nos. 3,845,770; 4,326,525; 4,902,514; and 5,616,345. Another embodiment for use in the methods disclosed herein employs transdermal delivery devices ("patches"). Such transdermal patches may be used to provide continuous or discontinuous infusion of the compounds or pharmaceutically acceptable salts thereof in controlled amounts. The construction and use of transdermal patches for the delivery of pharmaceutical agents is described, for example, U.S. Pat. Nos. 5,023,252, 4,992,445 and 5,001,139. Such patches may be constructed for continuous, pulsatile, or on-demand delivery of pharmaceutical agents.

The compositions disclosed herein may be formulated in a unit dosage form. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient (e.g., a tablet, capsule, ampoule). The compounds or pharmaceutically acceptable salt thereof are generally administered in a pharmaceutically effective amount. Preferably, for oral administration, each dosage unit contains from about 1 mg to about 2 g of a compound or pharmaceutically acceptable salt thereof as described herein, and for parenteral administration, preferably from about 0.1 to about 700 mg of a compound or pharmaceutically acceptable salt thereof as described herein. It will be understood, however, that the amount of the compound or pharmaceutically acceptable salt thereof actually administered usually will be determined by a physician, in the light of the relevant circumstances, including the condition to be treated, the chosen route of administration, the actual compound or pharmaceutically acceptable salt thereof administered and its relative activity, the age, weight, and response of the individual patient, the severity of the patient's symptoms, and the like.

For preparing solid compositions such as tablets, the principal active ingredient may be mixed with a pharmaceutical excipient to form a solid preformulation composition containing a homogeneous mixture of a compound or pharmaceutically acceptable salt thereof as disclosed herein. When referring to these preformulation compositions as homogeneous, it is meant that the active ingredient is dispersed evenly throughout the composition so that the composition may be readily subdivided into equally effective unit dosage forms such as tablets, pills and capsules.

The tablets or pills disclosed herein may be coated or otherwise compounded to provide a dosage form affording the advantage of prolonged action, or to protect from the acid conditions of the stomach. For example, the tablet or pill can comprise an inner dosage and an outer dosage component, the latter being in the form of an envelope over the former. The two components can be separated by an enteric layer that serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described herein. In certain embodiments, the compositions are administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a facemask tent, or intermittent positive pressure breathing machine. Solution, suspension, or powder compositions may be administered, such as orally or nasally, from devices that deliver the formulation in an appropriate manner.

In some embodiments, there is provided a pharmaceutical composition comprising a compound, or pharmaceutically acceptable salt thereof, as disclosed herein and a pharmaceutically acceptable carrier.

### EXAMPLES

In order that the embodiments described herein may be more fully understood, the following examples are set forth. The synthetic and biological examples described in this application are offered to illustrate the compounds, pharmaceutical compositions and methods provided herein and are not to be construed in any way as limiting their scope.

The compounds provided herein can be prepared from readily available starting materials using the following general methods and procedures. It will be appreciated that where typical or preferred process conditions (i.e., reaction temperatures, times, mole ratios of reactants, solvents, pressures, etc.) are given, other process conditions can also be used unless otherwise stated. Optimal reaction conditions may vary with the particular reactants or solvent used, but such conditions can be determined by one skilled in the art by routine optimization.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are described in the art. For example, numerous protecting groups, and their introduction and removal, are described in T. W. Greene and P. G. M. Wuts, Protecting Groups in Organic Synthesis, Second Edition, Wiley, New York, 1991, and references cited therein.

The compounds provided herein may be isolated and purified by known standard procedures. Such procedures include recrystallization, filtration, flash chromatography, trituration, high pressure liquid chromatography (HPLC), or supercritical fluid chromatography (SFC). Note that flash chromatography may either be performed manually or via an automated system. The compounds provided herein may be characterized by known standard procedures, such as nuclear magnetic resonance spectroscopy (NMR) or liquid chromatography mass spectrometry (LCMS). NMR chemical shifts are reported in part per million (ppm) and are generated using methods described in the art.

### List of abbreviations

- THF: tetrahydrofuran
- TFA: trifluoroacetic acid
- DMF: *N,N*-dimethylformamide
- DCM: dichloromethane
- ACN: acetonitrile
- EtOAc: ethyl acetate
- DIPEA: *N,N,*-diisopropylethylamine
- HATU: o-(7-azabenzotriazol-1-yl)-*N,N,N',N'*-tetramethyluronium hexafluorophosphate
- DCC: N,N'-dicyclohexylcarbodiimide
- DMSO: dimethyl sulfoxide
- EGTA: ethylene glycol-bis(β-aminoethyl ether)-*N,N,N',N'*-tetraacetic acid
- NMDG: *N*-methyl-D-glucamine
- HEPES: 4-(2-hydroxyethyl)piperazine-1-ethanesulfonic acid
- IC₅₀: half maximal inhibitory concentration
- TLC: thin layer chromatography
- LCMS: liquid chromatography-mass spectrometry
- HPLC: high-performance liquid chromatography
- SFC: supercritical fluid chromatography
- MS: mass spectrometry
- NMR: nuclear magnetic resonance

### Example 1. Synthesis of 2-(3-(2-cyclopropylpyridin-4-yl)-1,2,4-oxadiazol-5-yl)-1-(piperidin-1-yl)propan-1-one (Compound 1)

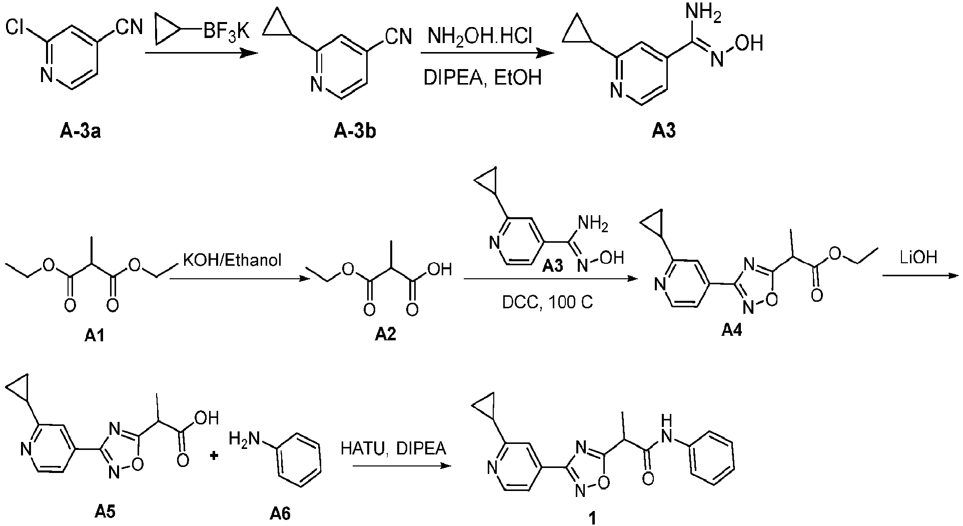

### Synthesis of 2-cyclopropylisonicotinonitrile (A-3b)

To a solution of 2-chloropyridine-4-carbonitrile (**A-3a**, 2.0 g, 14.4 mmol) in 1,4-dioxane (25 mL) was added potassium cyclopropyltrifluoroborate (6.41 g, 43.3 mmol) followed by K₂CO₃ (7.98 g, 57.7 mmol) and RuPhos (1.35 g, 2.89 mmol). The resulting mixture was degassed with N₂ gas for 10 minutes and Pd(OAc)₂ (324 mg, 1.44 mmol) was added. The mixture was stirred at 100 °C for 1 hour. The reaction mixture was cooled to room temperature and filtered through celite. The filtrate was concentrated under reduced pressure, and the product was purified by column chromatography on silica gel with 15% EtOAc/PE to afford compound **A-3b** (1.1 g, 7.6 mmol, 50% yield) as a solid. **LCMS:** 145.1 (M+H), Rt 1.87 min; Column: ZORBAX XDB C-18 (50 x 4.6 mm), 3.5 µm Mobile Phase: A: 0.1% TFA in water:ACN (95:5), B: 0.1% TFA in ACN; Flow Rate:1.5 mL/min.

### Synthesis of (Z)-2-cyclopropyl-N'-hydroxyisonicotinimidamide (A3)

To a solution of compound **A-3b** (450 mg, 3.1 mmol) in ethanol (15.0 mL) was added hydroxylamine hydrochloride (312 mg, 4.4 mmol) followed by DIPEA (1.49 mL, 8.99 mmol) at room temperature. The reaction mixture was heated at 80 °C for 5 hours. The reaction mixture was cooled to room temperature and concentrated under reduced pressure. The residue was treated with water (30 mL) followed by saturated sodium bicarbonate solution (20 mL) and extracted with ethyl acetate (2 x 25 mL). The organic layer was washed with brine (10 mL), dried over Na₂SO₄, and concentrated to afford compound **A3** (420 mg) as a solid.

### Synthesis of 3-ethoxy-2-methyl-3-oxo-propanoic acid (A2)

To a stirring solution of **A1** (5 g, 28.7 mmol) in ethanol (50 mL) was added KOH (2.4 g, 43.06 mmol) at room temperature and stirred for 24 hours. The ethanol was removed under reduced pressure. The product was dissolved in water, acidified with dilute HCl, and extracted with ethyl acetate. The combined organic solvent was dried over anhydrous Na₂SO₄ and filtered, and the filtrate was concentrated to give **A2** (3.1 g, 21.21 mmol, 74 % yield) as an oil which was used in the next step without further purification.

### Synthesis of ethyl 2-[3-(2-cyclopropyl-4-pyridyl)-1,2,4-oxadiazol-5-yl]propanoate (A4)

To a stirred solution of **A3** (1 g, 5.64 mmol) and **A2** (989.66 mg, 6.77 mmol) in 1,4-Dioxane (10 mL) was added DCC (1278.78 mg, 6.21 mmol) at room temperature and stirred at 100 °C for 16 hours. The solvent was removed under reduced pressure, and the mixture was diluted with ethyl acetate. The resulting mixture was filtered. The filtrate was evaporated, and the product was purified by column chromatography to give **A4** (1,1 g, 3.83 mmol, 68 % yield) as an oil.

### Synthesis of 2-[3-(2-cyclopropyl-4-pyridyl)-1,2,4-oxadiazol-5-yl]propanoic acid (A5)

To a stirred solution of **A4** (1 g, 3.4 8mmol) in THF (5 mL) was added lithium hydroxide (166.72 mg, 6.96 mmol) in water (1 mL) and stirred at room temperature for 12 hours. The solvent was removed under reduced pressure, acidified with dilute HCl, and then extracted with ethyl acetate. The combined organic layer was dried over anhydrous Na₂SO₄, filtered, and concentrated to afford **A5** (700 mg, 2.7 mmol, 77 % yield) as an oil.

### Synthesis of 2-[3-(2-cyclopropyl-4-pyridyl)-1,2,4-oxadiazol-5-yl]-N-phenyl-propanamide (1)

To a stirred solution of A5 (200 mg, 0.77 mmol) and aniline (0.11 mL, 1.16 mmol) in DCM (10 mL) was added DIPEA (0.34 mL, 1.93 mmol) and HATU (439.98 mg, 1.16 mmol) at room temperature and stirred for 4 hours. The reaction mixture was diluted with water and extracted with DCM (3 times). The combined organic layer was dried over anhydrous Na₂SO₄ and filtered, and the filtrate was evaporated to give the product, which was purified by prep-HPLC to give **1** (110 mg, 0.323 mmol, 42 % yield) as a solid. **HPLC:** Rt 8.30 min, 99.8%; Column: X-Select CSH C18 (4.6 x 150) mm, 5 µm; Mobile phase: A: 0.1% Formic acid in water: ACN (95:05), B: ACN; Flow Rate: 1.0 mL/min. **LCMS** : 334.7 (M+H), Rt 1.905 min, Column: X-select CSH C18 (3 x 50) mm, 2.5 µm. **¹H NMR (400 MHz, DMSO-d6)** δ_{H} = 10.51 (s, 1H), 8.60 (d, 1H), 7.87 (s, 1H), 7.67-7.65 (m, 1H), 7.60 (d, 2H), 7.34 (t, 2H), 7.09 (t, 1H), 4.43 (q, 1H), 2.32-2.26 (m, 1H), 1.69 (d, 3H), 1.03-0.95 (m, 4H).

### Example 2. Synthesis of N-benzyl-2-(3-(2-cyclopropylpyridin-4-yl)-1,2,4-oxadiazol-5-yl)propanamide (Compound 2)

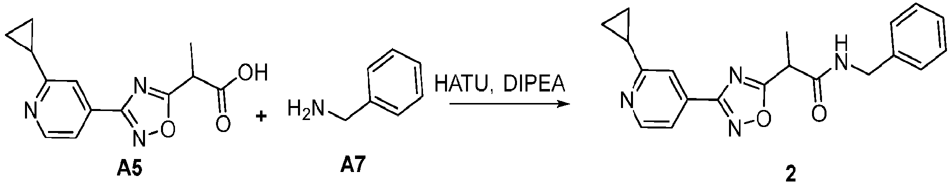

To a stirred solution of **A5** (80.mg, 0.31 mmol) and benzylamine (0.05 mL, 0.46 mmol) in DCM (10 mL) was added DIPEA (0.13 mL, 0.77 mmol) and HATU (175.99 mg, 0.46 mmol) at room temperature and stirred for 4 hours. The reaction mixture was quenched with water and extracted with DCM. The combined organic layer was dried over anhydrous Na₂SO₄, filtered, and concentrated to give the product, which was purified by prep-HPLC to give 2 (40 mg, 0.113 mmol, 36 % yield) as a solid. **HPLC:** Rt 7.720 min, 98%; Column: X-Select CSH C18 (4.6 x 150) mm, 3.5 µm; Mobile phase: A: 0.1% Formic acid in water: ACN (95:05), B: ACN; Flow Rate: 1.0 mL/min. **LCMS :** 349.15 (M+H), Rt 2.024 min, Column: X-select CSH C18 (3 x 50) mm, 2.5 µm. **¹H NMR** (**400 MHz, DMSO-d6**) δ_{H} = 8.96-8.90 (m, 1H), 8.62 (d, 1H), 7.87 (s, 1H), 7.68 (d, 1H), 7.36-7.22 (m, 5H), 4.35-4.27 (m, 3H), 2.32-2.28 (m, 1H), 1.62 (d, 3H), 1.03-0.99 (m, 4H).

### Example 3. Synthesis of (2R)-N-cyclohexyl-2-[3-(2-cyclopropyl-4-pyridyl)-1,2,4-oxadiazol-5-yl]propanamide (Compound 3) and (2S)-N-cyclohexyl-2-[3-(2-cyclopropyl-4-pyridyl)-1,2,4-oxadiazol-5-yl]propenamide (Compound 4). Note that stereochemistry is randomly assigned

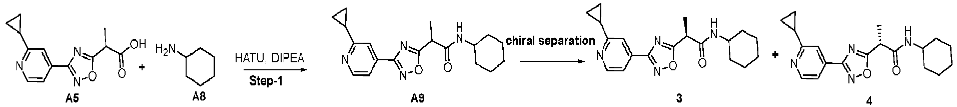

To a stirred solution of **A5** (80 mg, 0.31 mmol) and cyclohexanamine (0.05 mL, 0.46 mmol) in DCM (10 mL) was added DIPEA (0.13 mL, 0.77 mmol) and HATU (175.99 mg, 0.46 mmol) at room temperature and stirred at room temperature for 4 hours. The reaction mixture was quenched with water and extracted with DCM. The combined organic layer was dried over anhydrous Na₂SO₄, filtered, and concentrated. The product was purified by prep-HPLC to give **A9,** which was separated by chiral column chromatography to give **3** (20 mg, 0.058 mmol, 19 % yield) and **4** (20 mg, 0.059 mmol, 19 % yield) as solids. The prep HPLC purification was carried out using SFC. The conditions were: column: YMC CHIRAL AMYLOSE-SA (250 x 30 mm, 5 um) - Mobile Phase: A) n-Hexane+0.1% TFA B) EtOH, Isocratic: 15% B; Wavelength: 292 nm, Flow: 30 mL/min.

**3: HPLC:** Rt 8.421 min, 98.3%; Column: X-Select CSH C18 (4.6 x 150) mm, 3.5 µm; Mobile phase: A: 0.1% Formic acid in water: ACN (95:05), B: ACN; Flow Rate: 1.0 mL/min. **LCMS** : 341.0 (M+H), Rt 1.991 min, Column: X-select CSH C18 (3 x 50) mm, 2.5 µm. **¹H NMR** (**400 MHz, DMSO-d6**) δ_{H} = 8.61 (d, 1H), 8.29 (d, 1H), 7.85 (s, 1H), 7.66 (d, 1H), 4.17-4.15 (m, 1H), 3.60-3.50 (m, 1H), 2.30- 2.28 (m, 1H), 1.77-1.65 (m, 4H), 1.56 (d, 3H), 1.28-1.14 (m, 6H), 1.02-0.98 (m, 4H). **Chiral method:** Rt 5.408 min, 98.7%; column: YMC CHIRAL AMYLOSE-SA (250x4.6mm,5u), - Mobile Phase: A) n-Hexane+0.1% TFA B) EtOH, Isocratic: 15% B; Wavelength: 292 nm, Flow: 1.0 mL/min.

**4: HPLC:** Rt 8.313 min, 99.7%; Column: X-Select CSH C18 (4.6 x 150) mm, 5 µm; Mobile phase: A: 0.1% Formic acid in water: ACN (95:05), B: ACN; Flow Rate: 1.0 mL/min. **LCMS** : 341.0 (M+H), Rt 1.978 min, Column: X-select CSH C18 (3 x 50) mm, 2.5 µm. **¹H NMR** (**400 MHz, DMSO-d6**) δ_{H} = 8.61 (d, 1H), 8.29 (d, 1H), 7.85 (s, 1H), 7.66 (d, 1H), 4.17-4.15 (m, 1H), 3.60-3.50 (m, 1H), 2.30- 2.28 (m, 1H), 1.77-1.65 (m, 4H), 1.56 (d, 3H), 1.28-1.14 (m, 6H), 1.02-0.98 (m, 4H). **Chiral method:** Rt 5.174 min + Rt 5.730 min, 50.253+49.565%; column: YMC CHIRAL AMYLOSE-SA (250 x 4.6 mm, 5 um), - Mobile Phase: A) n-Hexane+0.1% TFA B) EtOH, Isocratic: 15% B; Wavelength: 292 nm, Flow: 1.0 mL/min.

### Example 4. Synthesis of 2-(3-(2-cyclopropylpyridin-4-yl)-1,2,4-oxadiazol-5-yl)-1-(piperidin-1-yl)propan-1-one (Compound 5)

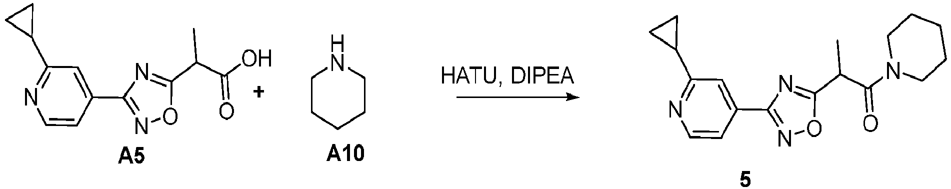

To a stirred solution of **A5** (200 mg, 0.77 mmol) and piperidine (0.11 mL, 1.16 mmol) in DCM (10 mL) was added DIPEA (0.34 mL, 1.93 mmol) and HATU (439.98 mg, 1.16 mmol) at room temperature and stirred for 4 hours. The reaction mixture was quenched with water and extracted with DCM. The combined organic layer was dried over anhydrous Na₂SO₄, filtered, and concentrated. The product was purified by prep-HPLC to give 5 (75 mg, 0.22 mmol, 29 % yield) as a solid. **HPLC:** Rt 7.629 min, 98.9%; Column: X-Select CSH C18 (4.6 x 150) mm, 3.5 µm; Mobile phase: A: 0.1% Formic acid in water: ACN (95:05), B: ACN; Flow Rate: 1.0 mL/min. **LCMS :** 326.8 (M+H), Rt 1.835 min, Column: X-select CSH C18 (3 x 50) mm, 2.5 µm. **¹H NMR** (**400 MHz, DMSO-d6**) δ_{H} = 8.63 (d, 1H), 7.88 (s, 1H), 7.73-7.70 (m, 1H), 4.84 (d, 1H), 3.60-3.39 (m, 4H), 2.35-2.28 (m, 1H), 1.62-1.45 (m, 9H), 1.09-0.99 (m, 4H).

### Example 5. Synthesis of (R)-2-(3-(2-cyclopropylpyridin-4-yl)-1,2,4-oxadiazol-5-yl)-1-(piperidin-1-yl)propan-1-one (Compound 6) and (S)-2-(3-(2-cyclopropylpyridin-4-yl)-1,2,4-oxadiazol-5-yl)-1-(piperidin-1-yl)propan-1-one (Compound 7):

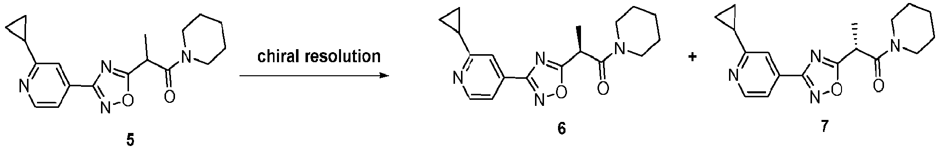

### Stereochemistry is randomly assigned

Compound **5** (75 mg) was separated by chiral-HPLC to give two enantiomers **6** (10 mg, 0.0304 mmol, 13 % yield) and **7** (10 mg, 0.0306 mmol, 13 % yield) as oils. The purification was performed by chiral HPLC separation method using Column: Chiralpak IG (250 X 30 5µm), Mobile phase: B: Methanol and A: CO₂. The gradient run was % of B: 30-35 % B 4 min, 35-40 % B 4 min, 40-45 % B 3 min, 40-40 % B 3 min, 40-45 % B 2 min, 45-50 %B 2 min, 50-50 %B 5 min. The flow rate employed was 80 ml/min. **Note: the absolute stereochemistry was randomly assigned. Compound 6 HPLC:** Rt 7.911 min, 99.2%; Column: X-Select CSH C18 (4.6 X 150) mm, 5 µm; Mobile phase: A: 0.1% Formic acid in water: ACN (95:05), B: ACN; Flow Rate: 1.0 mL/min. LCMS : 327.5 (M+H), Rt 1.718 min, Column: X-select CSH (3*50) mm, 2.5 µm. **¹H NMR (400 MHz, DMSO-d6)** δ_{H} = 8.61 (d, 1H), 7.85 (s, 1H), 7.66 (d, 1H), 4.84-4.82 (m, 1H), 3.56-3.54 (m, 2H), 3.45-3.41 (m, 2H), 2.31-2.27 (m, 1H), 1.60-1.55 (m, 7H), 1.50-1.45 (m, 2H), 1.02-0.98 (m, 4H). **Chiral method:** Rt 5.039 min, 99.8%; column: DIACEL CHIRALPAK-IG (250x4.6mm,5u), - Mobile Phase: A) n-Hexane+0.1% Iso-propyl amine B) DCM: MeOH (1:1), Isocratic:50% B; Wavelength: 293 nm, Flow: 1.0 mL/min. **Compound 7 HPLC:** Rt 7.924 min, 99.9%; Column: X-Select CSH C18 (4.6 X 150) mm, 3.5 µm; Mobile phase: A: 0.1% Formic acid in water: ACN (95:05), B: ACN; Flow Rate: 1.0 mL/min. **LCMS** : 327.5 (M+H), Rt 1.715 min, Column: X-select CSH (3*50) mm, 2.5 µm. **¹H NMR (400 MHz, DMSO-d6)** δ_{H} = 8.61 (d, 1H), 7.86 (s, 1H), 7.66 (d, 1H), 4.86-4.81 (m, 1H), 3.56-3.41 (m, 4H), 2.32-2.26 (m, 1H), 1.60-1.54 (m, 7H), 1.50-1.45 (m, 2H), 1.02-0.98 (m, 4H). **Chiral method:** Rt 7.182 min, 100%; column: DIACEL CHIRALPAK-IG (250x4.6mm,5u), - Mobile Phase: A) n-Hexane+0.1% Iso-propyl amine B) DCM: MeOH (1:1), Isocratic:50% B; Wavelength: 226 nm, Flow: 1.0 mL/min.

### Example 6. Synthesis of (R)-2-(3-(2-cyclopropylpyridin-4-yl)-1,2,4-oxadiazol-5-yl)-N-phenylpropanamide (Compound 8) and (S)-2-(3-(2-cyclopropylpyridin-4-yl)-1,2,4-oxadiazol-5-yl)-N-phenylpropanamide (Compound 9):

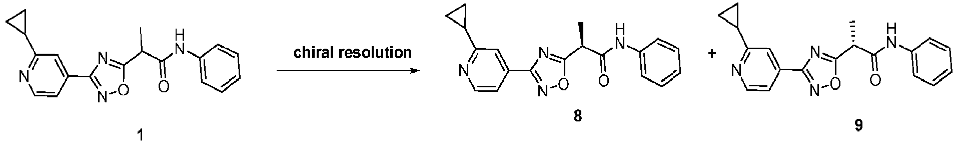

### Stereochemistry is randomly assigned

Compound **1** (110 mg) was separated by chiral-HPLC to give two enantiomers **8** (20 mg, 0.0597 mmol, 18% yield) and **9** (20 mg, 0.0597 mmol, 18 % yield) as solids. The purification was performed by chiral HPLC separation method using Column: Chiralpak IG (250 X 30 5µm), Mobile phase: B: Methanol and A: CO₂. The gradient run was % of B: 20-40 % B 3 min, 40-40 % B 6 min, 40-45 % B 3 min and 45-50 % B 3 min. The flow rate employed was 80 ml/min. **Note: the absolute stereochemistry was randomly assigned. Compound 8 HPLC:** Rt 8.338 min, 99.9%; Column: X-Select CSH C18 (4.6 X 150) mm, 3.5 µm; Mobile phase: A: 0.1% Formic acid in water: ACN (95:05), B: ACN; Flow Rate: 1.0 mL/min. **LCMS** : 335.05 (M+H), Rt 1.733 min, Column: X-select CSH (3*50) mm, 2.5 µm. **¹H NMR (400 MHz, DMSO-d6)** δ_{H} = 10.5 (s, 1H), 8.59 (d, 1H), 7.86 (s, 1H), 7.66 (d, 1H), 7.59 (d, 2H), 7.35-7.31 (m, 2H), 7.11-7.07 (m, 1H), 4.43 (q, 1H), 2.32-2.25 (m. 1H), 1.69 (d, 3H), 1.00-0.97 (m, 4H). **Chiral method:** Rt 13.626 min, 100%; column: DIACEL CHIRALPAK-IG (250x4.6mm,5u), - Mobile Phase: A) n-Hexane+0.1% Iso-propyl amine B) DCM: MeOH (1:1), Isocratic:20% B; Wavelength: 234 nm, Flow: 1.0 mL/min. **Compound 9 HPLC:** Rt 8.291 min, 99.9%; Column: X-Select CSH C18 (4.6 X 150) mm, 5 µm; Mobile phase: A: 0.1% Formic acid in water: ACN (95:05), B: ACN; Flow Rate: 1.0 mL/min. **LCMS** : 335.35 (M+H), Rt 1.746 min, Column: X-select CSH (3*50) mm, 2.5 µm. **¹H NMR (400 MHz, DMSO-d6)** δ_{H} = 10.5 (s, 1H), 8.59 (d, 1H), 7.87 (s, 1H), 7.66 (d, 1H), 7.59 (d, 2H), 7.35-7.31 (m, 2H), 7.11-7.07 (m, 1H), 4.43 (q, 1H), 2.32-2.26 (m, 1H), 1.69 (d, 3H), 1.00-0.97 (m, 4H). **Chiral method:** Rt 13.690 min, 100%; column: DIACEL CHIRALPAK-IG (250x4.6mm,5u), - Mobile Phase: A) n-Hexane+0.1% Iso-propyl amine B) DCM: MeOH (1:1), Isocratic:20% B; Wavelength: 234 nm, Flow: 1.0 mL/min.

### Example 7. Synthesis of -[3-(2-cyclopropyl-4-pyridyl)-1,2,4-oxadiazol-5-yl]-N-methyl-N-phenyl-propanamide (Compound 10)

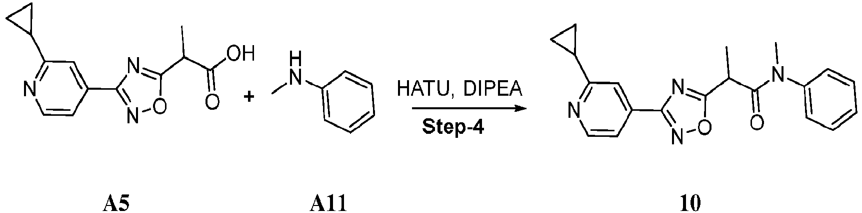

To a stirred solution of **A5** (100 mg, 0.39 mmol) and *N*-methylaniline (0.11 mL, 0.58 mmol) in DCM (10 mL) was added DIPEA (0.17 mL, 0.96 mmol) and HATU (219.99 mg, 0.58 mmol) at room temperature and stirred for 4 hours. The reaction mixture was quenched with water and extracted with DCM (3 times). The combined organic layer was dried over anhydrous Na₂SO₄ and filtered off, and the filtrate was concentrated. The product was purified by prep-HPLC to give **10** (40 mg,0.114 mmol, 29 % yield) as a solid. **HPLC:** Rt 8.559 min, 99.4%; Column: X-Select CSH C18 (4.6 X 150) mm, 5 µm; Mobile phase: A: 0.1% Formic acid in water: ACN (95:05), B: ACN; Flow Rate: 1.0 mL/min. **LCMS:** 349.5 (M+H), Rt 1.854 min, Column: X-select CSH C18 (3*50) mm, 2.5 µm. **¹H NMR (400 MHz, DMSO-d6)** δ_{H} = 8.59 (d, 1H), 7.81 (s, 1H), 7.60 (d, 1H), 7.50-7.47 (m, 2H), 7.42-7.40 (m, 3H), 4.19 (q, 1H), 3.22 (s, 3H), 2.32-2.25 (m, 1H), 1.48 (d, 3H), 1.02-0.97 (m, 4H).

### Example 8. Synthesis of 2-(3-(2-cyclopropylpyridin-4-yl)-1,2,4-oxadiazol-5-yl)-N-((S)-1-phenylethyl)propanamide (Compound 11):

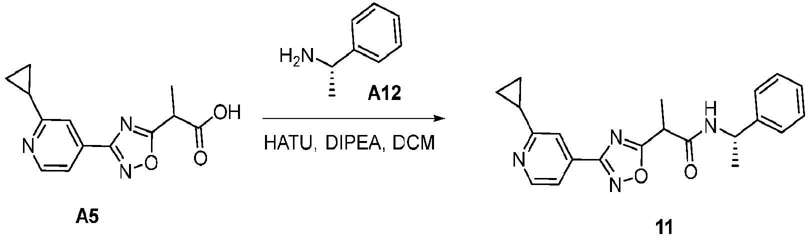

To a stirred solution of **A5** (150. mg, 0.58 mmol) and **A12** (0.11 mL, 0.87 mmol) in DCM (10 mL) was added DIPEA (0.25 mL, 1.45 mmol) followed by HATU (329.98 mg, 0.87 mmol) at room temperature. The reaction mixture was stirred at room temperature for 4 hours. The reaction mixture was diluted with water and extracted with DCM. The organic layer was separated, dried over anhydrous sodium sulphate, filtered, and evaporated under reduced pressure to give a product, which was purified by prep-HPLC to give **11** (60 mg, 0.166 mmol, 29% yield) as a solid. **HPLC:** Rt 8.264 min, (44.14%) + Rt 8.331 min, (55.86%); Column: X-Select CSH C18 (4.6 X 150) mm, 3.5 µm; Mobile phase: A: 0.1% Formic acid in water: ACN (95:05), B: ACN; Flow Rate: 1.0 mL/min. **LCMS** : 363.15 (M+H), Rt 1.94 min, Column: X-select CSH (3*50) mm, 2.5 µm. **¹H NMR (400 MHz, DMSO-d6)** δ_{H} = 8.87 (d, 1H), 8.60 (d, 1H), 7.84 (d, 1H), 7.66-7.62 (m, 1H), 7.33-7.22 (m, 5H), 4.93-4.89 (m, 1H), 4.27-4.25 (m, 1H), 2.30-2.27 (m, 1H), 1.59 (dd, 3H), 1.38 (d, 3H), 1.01-0.97 (m, 4H).

### Example 9. Synthesis of 2-(3-(2-cyclopropylpyridin-4-yl)-1,2,4-oxadiazol-5-yl)-N-((R)-1-phenylethyl)propanamide (Compound 12):

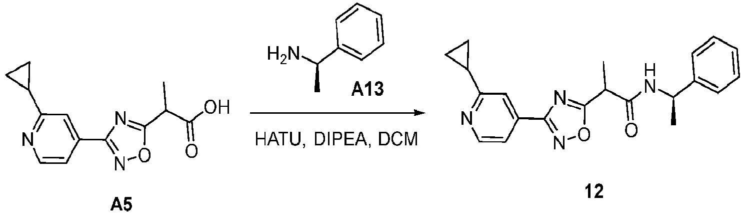

To a stirred solution of **A5** (150. mg, 0.58 mmol) and **A13** (0.11 mL, 0.87 mmol) in DCM (10 mL) was added DIPEA (0.25 mL, 1.45 mmol) followed by HATU (329.98 mg, 0.87 mmol) at room temperature. The reaction mixture was stirred at room temperature for 4 hours. The reaction mixture was diluted with water and extracted with DCM. The organic layer was separated, dried over anhydrous sodium sulphate, filtered, and evaporated under reduced pressure to give the product, which was purified by prep-HPLC to give **12** (70 mg, 0.19 mmol, 33 % yield) as a solid. **HPLC:** Rt 7.995 min, 46.24%+8.067 min, 53.43%; Column: X-Select CSH C18 (4.6 X 150) mm, 3.5 µm; Mobile phase: A: 0.1% Formic acid in water: ACN (95:05), B: ACN; Flow Rate: 1.0 mL/min. **LCMS** : 363.15 (M+H), Rt 1.942 min, Column: X-select CSH (3*50) mm, 2.5 µm. **¹H NMR (400 MHz, DMSO-d6)** δ_{H} = 8.87 (d, 1H), 8.67 (d, 1H), 7.92 (d, 1H), 7.82-7.77 (m, 1H), 7.35-7.28 (m, 4H), 7.27-7.23 (m, 1H), 4.94-4.89 (m, 1H), 4.29 (q, 1H), 2.37-2.31 (m, 1H), 1.60 (dd, 3H), 1.38 (d, 3H), 1.14-1.04 (m, 4H).

### Example 10. Efficacy of exemplary compounds in the inhibition of KCNT1

### KCNT1-WT-Basal - Patch Clamp Assay

Inhibition of KCNT1 (KNa1.1, Slack) was evaluated using a tetracycline inducible cell line (HEK-TREX). Currents were recorded using the SyncroPatch 384PE automated, patch clamp system. Pulse generation and data collection were performed with PatchController384 V1.3.0 and DataController384 V1.2.1 (Nanion Technologies). The access resistance and apparent membrane capacitance were estimated using built-in protocols. Current were recorded in perforated patch mode (10 µM escin) from a population of cells. The cells were lifted, triturated, and resuspended at 800,000 cells/ml. The cells were allowed to recover in the cell hotel prior to experimentation. Currents were recorded at room temperature. The external solution contained the following (in mM): NaCl 105, NMDG 40, KCl 4, MgCl₂ 1, CaCl₂ 5 and HEPES 10 (pH = 7.4, Osmolarity ~300 mOsm). The extracellular solution was used as the wash, reference and compound delivery solution. The internal solution contained the following (in mM): NaCl 70, KF 70, KCl 10, EGTA 5, HEPES 5 and Escin 0.01 (pH = 7.2, Osmolarity ~295 mOsm). Escin is made at a 5mM stock in water, aliquoted, and stored at -20°C. The compound plate was created at 2x concentrated in the extracellular solution. The compound was diluted to 1:2 when added to the recording well. The amount of DMSO in the extracellular solution was held constant at the level used for the highest tested concentration. A holding potential of -80 mV with a 100ms step to 0mV was used. Mean current was measured during the step to 0 mV. 100 µM Bepridil was used to completely inhibit KCNT1 current to allow for offline subtraction of non-KCNT1 current. The average mean current from 3 sweeps was calculated and the % inhibition of each compound was calculated. The % Inhibition as a function of the compound concentration was fit with a Hill equation to derive IC₅₀, slope, min and max parameters. If KCNT1 inhibition was less than 50% at the highest tested concentration or if an IC₅₀ could not be calculated, then a percent inhibition was reported in place of the IC₅₀. Results from this assay are summarized in Table 1 below. In this table, "A" indicates IC₅₀ of less than or equal to1 µM; "B" indicates inhibition of between 1 µM to 20 µM; and "C" indicates inhibition of greater than or equal to 20 µM.

**Table 1**

| Compound No. | KCNT1 WT IC₅₀ (µM) |
|---|---|
| 1 | A |
| 2 | A |
| 3 | A |
| 4 | A |
| 5 | B |
| 6 | B |
| 7 | C |
| 8 | A |
| 9 | A |
| 10 | B |
| 11 | A |
| 12 | A |

### Equivalents and Scope

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure includes embodiments in which more than one, or all of the group members are present in, employed in, or otherwise relevant to a given product or process.

Furthermore, the embodiments encompass all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, e.g., in Markush group format, each subgroup of the elements is also disclosed, and any element(s) can be removed from the group. It should it be understood that, in general, where the embodiment, or aspects of the embodiment, is/are referred to as comprising particular elements and/or features, certain embodiments or aspects consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the disclosure, to the tenth of the unit of the lower limit of the range, unless the context clearly dictates otherwise.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims. Those of ordinary skill in the art will appreciate that various changes and modifications to this description may be made without departing from the scope of the present disclosure.

## Claims

1. A compound of Formula I having an oxadiazole core: 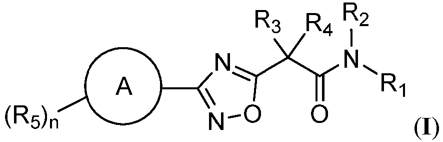 or a pharmaceutically acceptable salt thereof, wherein
ring A is a 5-6 membered heteroaryl;
R₁ is chosen from a C₁₋₆alkyl, a C₃₋₈cycloalkyl, a phenyl, a 3-7 membered heterocyclyl, or a 5-6 membered heteroaryl, wherein the C₁₋₆alkyl, C₃₋₈cycloalkyl, phenyl, 3-7 membered heterocyclyl, or 5-6 membered heteroaryl optionally comprises one or more R₆ substituents;
R₂ is chosen from hydrogen or a C₁₋₆alkyl; or
R₁ and R₂ are taken together with the nitrogen attached to R₁ and R₂ to form a 3-7 membered heterocyclyl optionally comprising one or more R₆ substituents;
R₃ is chosen from hydrogen or a C₁₋₆alkyl optionally comprising one or more substituents chosen from a halogen, a C₁₋₆haloalkyl, a C₁₋₆alkoxy, or a C₁₋₆haloalkoxy;
R₄ is a C₁₋₆alkyl optionally comprising one or more substituents chosen from a halogen, a C₁₋₆haloalkyl, a C₁₋₆alkoxy, or a C₁₋₆haloalkoxy; or
R₃ and R₄ are taken together with the carbon attached to R₃ and R₄ to form a C₃₋₈cycloalkylene or 3-7 membered heterocycloalkylene;
R₅ is each independently chosen from a halogen, a C₁₋₆alkyl, a C₁₋₆haloalkyl, a C₁₋₆alkoxy, a C₁₋₆haloalkoxy, a -N(R₇)₂, a C₁₋₆alkylene-C₁₋₆alkoxy, or a C₃₋₈cycloalkyl;
R₆ is each independently chosen from a halogen, a C₁₋₆alkyl, a C₁₋₆haloalkyl, a C₁₋₆alkoxy, a C₁₋₆haloalkoxy, a C₃₋₈cycloalkyl, a 3-7 membered heterocyclyl, a 5-6 membered heteroaryl, or a phenyl;
each R₇ is independently chosen from hydrogen, a C₁₋₆alkyl, or a -(C₁₋₆alkylene)-OH, or the two R₇ are taken together with the nitrogen atom attached to the two R₇ to form a heterocycle optionally comprising one or more substituents chosen from a halogen and -OH; and
n is 0, 1, 2, or 3.

2. The compound of claim 1, wherein the compound is a compound of Formula I-a: 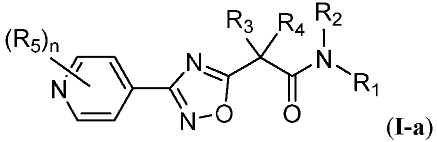 or a pharmaceutically acceptable salt thereof.

3. The compound of claim 1 or 2, wherein the compound is a compound of Formula I-**a1**, Formula I-a2, Formula 1-a3, Formula I-a4, or Formula I-a5: 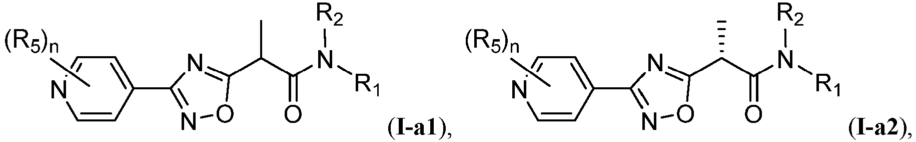 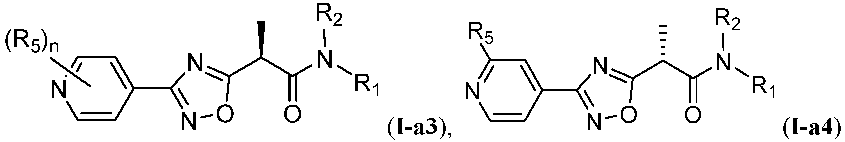 or 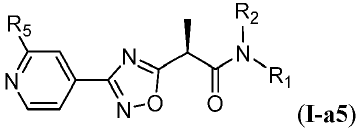 or a pharmaceutically acceptable salt thereof.

4. The compound of any one of claims 1-3, wherein R₁ is chosen from a C₁₋₆alkyl, a C₃₋₈cycloalkyl, or a phenyl, each optionally comprising one R₆ substituent, preferably wherein R₁ is a C₁₋₆alkyl comprising one R₆ substituent, such as a phenyl substituent.

5. The compound of any one of claims 1-4, wherein R₂ is hydrogen.

6. The compound of any one of claims 1, 2, and 4-5, wherein R₃ is a C₁₋₆alkyl, such as a methyl.

7. The compound of claim 1, wherein the compound is chosen from: 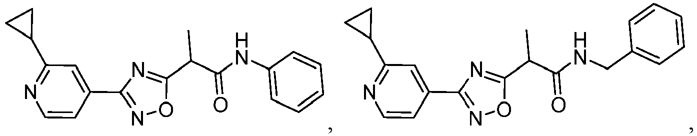 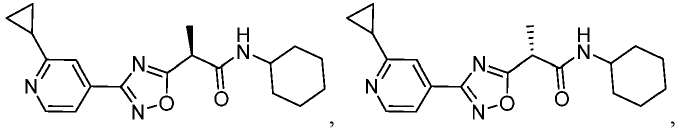 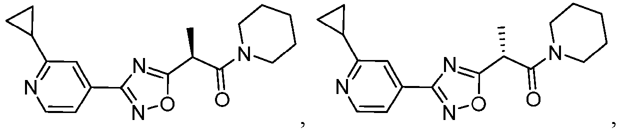 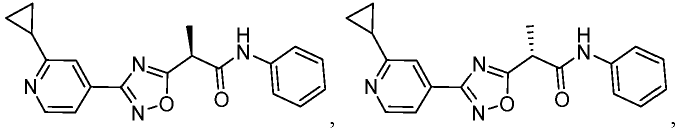 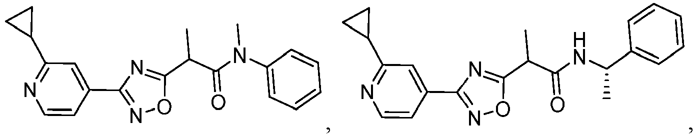 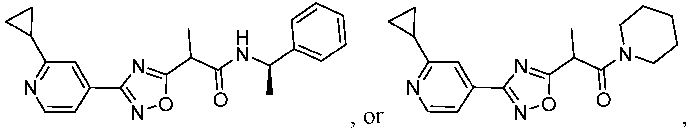 or a pharmaceutically acceptable salt thereof.

8. A pharmaceutical composition comprising a compound of any one of claims 1-7, or a pharmaceutically acceptable salt thereof, and a pharmaceutically acceptable excipient.

9. A compound of any one of claims 1-7 or a pharmaceutically acceptable salt thereof or a pharmaceutical composition of claim 8, for use in a method of treating a neurological disorder, a disorder associated with excessive neuronal excitability, or a disorder associated with a gain-of-function mutation of a gene (e.g., KCNT1).

10. The compound or a pharmaceutically acceptable salt thereof or a pharmaceutical composition for use according to claim 9, wherein the neurological disorder, the disorder associated with excessive neuronal excitability, or the disorder associated with a gain-of-function mutation of a gene (e.g., KCNT1) is epilepsy, an epilepsy syndrome, an encephalopathy, such as a genetic or pediatric epilepsy or a genetic or pediatric epilepsy syndrome.

11. The compound or a pharmaceutically acceptable salt thereof or a pharmaceutical composition for use according to claim 9, wherein the neurological disorder, the disorder associated with excessive neuronal excitability, or the disorder associated with a gain-of-function mutation of a gene (e.g., KCNT1) is chosen from epilepsy and other encephalopathies (e.g., malignant migrating focal seizures of infancy (MMFSI) or epilepsy of infancy with migrating focal seizures (EIMFS), autosomal dominant nocturnal frontal lobe epilepsy (ADNFLE), West syndrome, infantile spasms, epileptic encephalopathy, focal epilepsy, Ohtahara syndrome, developmental and epileptic encephalopathy, Lennox-Gastaut syndrome, seizures (e.g., Generalized tonic clonic seizures, Asymmetric Tonic Seizures), leukodystrophy, leukoencephalopathy, intellectual disability, Multifocal Epilepsy, Drug resistant epilepsy, Temporal lobe epilepsy, or cerebellar ataxia).

12. The compound or a pharmaceutically acceptable salt thereof or a pharmaceutical composition for use according to claim 9, wherein the neurological disorder, the disorder associated with excessive neuronal excitability, or the disorder associated with a gain-of-function mutation of a gene (e.g., KCNT1) is chosen from a cardiac dysfunction, cardiac arrhythmia, Brugada syndrome, myocardial infarction, pain and related conditions (e.g. neuropathic pain, acute/chronic pain, migraine), a muscle disorder (e.g., myotonia, neuromyotonia, cramp muscle spasms, spasticity), itch and pruritis, ataxia, cerebellar ataxias, psychiatric disorders (e.g., major depression, anxiety, bipolar disorder, schizophrenia), a learning disorder, Fragile X, neuronal plasticity, or an autism spectrum disorder.

13. The compound or a pharmaceutically acceptable salt thereof or a pharmaceutical composition for use according to claim 9, wherein the neurological disorder, the disorder associated with excessive neuronal excitability, or the disorder associated with a gain-of-function mutation of a gene (e.g., KCNT1) is chosen from epileptic encephalopathy with SCN1A, SCN2A, and/or SCN8A mutations, early infantile epileptic encephalopathy, Dravet syndrome, Dravet syndrome with SCN1A mutation, generalized epilepsy with febrile seizures, intractable childhood epilepsy with generalized tonic-clonic seizures, infantile spasms, benign familial neonatal-infantile seizures, SCN2A epileptic encephalopathy, focal epilepsy with SCN3A mutation, cryptogenic pediatric partial epilepsy with SCN3A mutation, SCN8A epileptic encephalopathy, Rasmussen encephalitis, malignant migrating partial seizures of infancy, autosomal dominant nocturnal frontal lobe epilepsy, KCNQ2 epileptic encephalopathy, or KCNT1 epileptic encephalopathy.

## Patentansprüche

1. Verbindung der Formel I mit einem Oxadiazolkern: 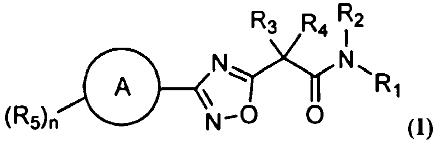 oder ein pharmazeutisch unbedenkliches Salz davon, wobei
Ring A ein 5-6-gliedriger Heteroarylring ist;
R₁ aus einem C₁₋₆-Alkyl, einem C₃₋₈-Cycloalkyl, einem Phenyl, einem 3-7-gliedrigen Heterocyclyl oder einem 5-6-gliedrigen Heteroaryl ausgewählt ist, wobei das C₁₋₆-Alkyl, C₃₋₈-Cycloalkyl, Phenyl, 3-7-gliedrige Heterocyclyl oder 5-6-gliedrige Heteroaryl optional einen oder mehrere R₆-Substituenten umfasst;
R₂ aus Wasserstoff oder einem C₁₋₆-Alkyl ausgewählt ist; oder
R₁ und R₂ zusammen mit dem an R₁ und R₂ angelagerten Stickstoff genommen werden, um ein 3-7-gliedriges Heterocyclyl auszubilden, das optional einen oder mehrere R₆-Substituenten umfasst;
R₃ aus Wasserstoff oder einem C₁₋₆-Alkyl ausgewählt ist, das optional einen oder mehrere Substituenten umfasst, ausgewählt aus einem Halogen, einem C₁₋₆-Haloalkyl, einem C₁₋₆-Alkoxy oder einem C₁₋₆-Haloalkoxy;
R₄ ein C₁₋₆-Alkyl ist, das optional einen oder mehrere Substituenten umfasst, ausgewählt aus einem Halogen, einem C₁₋₆-Haloalkyl, einem C₁₋₆-Alkoxy oder einem C₁₋₆-Haloalkoxy; oder
R₃ und R₄ zusammen mit dem an R₃ und R₄ angelagerten Kohlenstoff genommen werden, um ein C₃₋₈-Cycloalkylen oder ein 3-7-gliedriges Heterocycloalkylen auszubilden;
R₅ jeweils unabhängig aus einem Halogen, einem C₁₋₆-Alkyl, einem C₁₋₆-Haloalkyl, einem C₁₋₆-Alkoxy, einem C₁₋₆-Haloalkoxy, einem -N(R₇)₂, einem C₁₋₆-Alkylen-C₁₋₆-alkoxy oder einem C₃₋₈-Cycloalkyl ausgewählt ist;
R₆ unabhängig aus Halogen, einem C₁₋₆-Alkyl, einem C₁₋₆-Haloalkyl, einem C₁₋₆-Alkoxy, einem C₁₋₆-Haloalkoxy, einem C₃₋₈-Cycloalkyl, einem 3-7-gliedrigem Heterocyclyl, 5-6-gliedrigem Heteroaryl oder einem Phenyl ausgewählt ist;
jedes R₇ unabhängig aus Wasserstoff, einem C₁₋₆-Alkyl oder einem -(C₁₋₆-Alkylen)-OH ausgewählt ist oder die zwei R₇ zusammen mit dem an die zwei R₇ angelagerten Stickstoffatom genommen werden, um einen Heterocyclus auszubilden, der optional einen oder mehrere Substituenten umfasst, die aus Halogen und -OH ausgewählt sind; und
n 0, 1, 2 oder 3 ist.

2. Verbindung nach Anspruch 1, wobei die Verbindung eine Verbindung der Formel I-a: 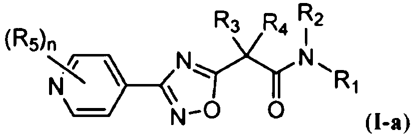 oder ein pharmazeutisch unbedenkliches Salz davon ist.

3. Verbindung nach Anspruch 1 oder 2, wobei die Verbindung eine Verbindung der Formel **I-a1**, Formel I-a2, Formel I-a3, Formel I-a4 oder Formel I-a5: 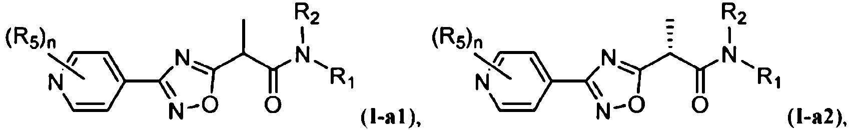 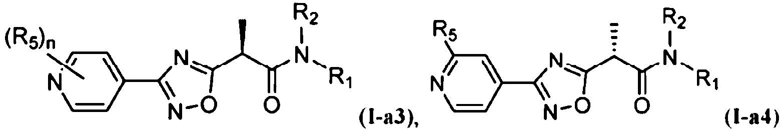 oder 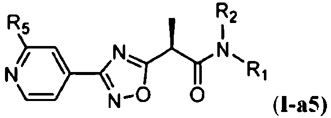 oder ein pharmazeutisch unbedenkliches Salz davon ist.

4. Verbindung nach einem der Ansprüche 1-3, wobei R₁ aus C₁₋₆-Alkyl, C₃₋₈-Cycloalkyl oder Phenyl ausgewählt ist, wobei jedes optional einen R₆-Substituenten umfasst, vorzugsweise wobei R₁ C₁₋₆-Alkyl ist, das einen R₆-Substituenten umfasst, wie einen Phenylsubstituenten.

5. Verbindung nach einem der Ansprüche 1-4, wobei R₂ Wasserstoff ist.

6. Verbindung nach einem der Ansprüche 1, 2 und 4-5, wobei R₃ C₁₋₆-Alkyl, wie Methyl, ist.

7. Verbindung nach Anspruch 1, wobei die Verbindung ausgewählt ist aus: 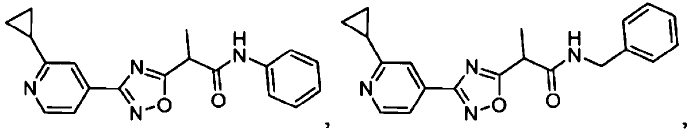 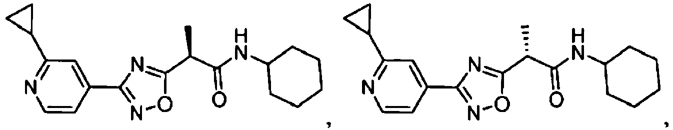 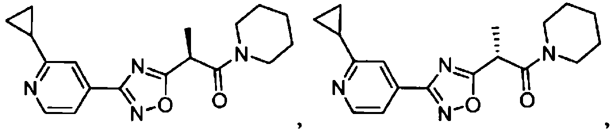 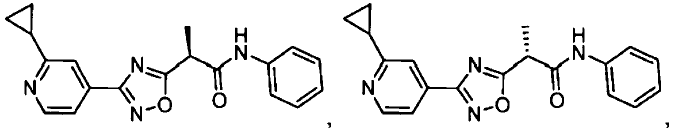 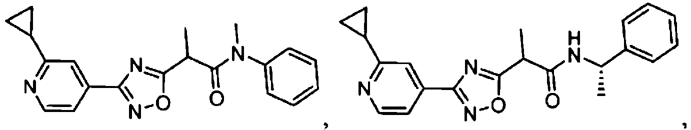 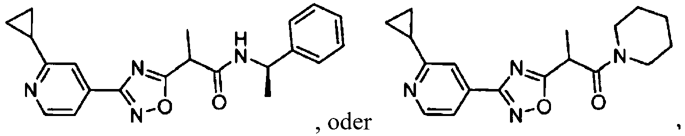 oder einem pharmazeutisch unbedenklichen Salz davon.

8. Pharmazeutische Zusammensetzung, die eine Verbindung nach einem der Ansprüche 1-7 oder ein pharmazeutisch unbedenkliches Salz davon und einen pharmazeutisch unbedenklichen Hilfsstoff umfasst.

9. Verbindung nach einem der Ansprüche 1-7 oder pharmazeutisch unbedenkliches Salz davon oder eine pharmazeutische Zusammensetzung nach Anspruch 8 für die Verwendung in einem Verfahren zum Behandeln einer neurologischen Erkrankung, einer Erkrankung, die mit einer übermäßigen neuronalen Erregbarkeit assoziiert ist, oder einer Erkrankung, die mit einer Funktionsgewinnmutation eines Gens (z. B. KCNT1) assoziiert ist.

10. Verbindung oder pharmazeutisch unbedenkliches Salz davon oder pharmazeutische Zusammensetzung für die Verwendung nach Anspruch 9, wobei die neurologische Erkrankung, die Erkrankung, die mit einer übermäßigen neuronalen Erregbarkeit assoziiert ist, oder die Erkrankung, die mit einer Funktionsgewinnmutation eines Gens (z. B. KCNT1) assoziiert ist, Epilepsie, ein Epilepsiesyndrom, eine Enzephalopathie, wie eine genetische oder pädiatrische Epilepsie oder ein genetisches oder pädiatrisches Epilepsiesyndrom ist.

11. Verbindung oder pharmazeutisch unbedenkliches Salz davon oder pharmazeutische Zusammensetzung für die Verwendung nach Anspruch 9, wobei die neurologische Erkrankung, die Erkrankung, die mit übermäßiger neuronaler Erregbarkeit assoziiert ist, oder die Erkrankung, die mit einer Funktionsgewinnmutation eines Gens (z. B. KCNT1) assoziiert ist, aus Epilepsie und anderen Enzephalopathien (z. B. malignen migrierenden fokalen Anfällen im Säuglingsalter (MMFSI) oder Epilepsie im Säuglingsalter mit migrierenden fokalen Anfällen (EIMFS), autosomal-dominanter nächtlicher Frontallappenepilepsie (ADNFLE), West-Syndrom, infantilen Spasmen, epileptischer Enzephalopathie, fokaler Epilepsie, Ohtahara-Syndrom, Entwicklungs- und epileptischer Enzephalopathie, Lennox-Gastaut-Syndrom, Anfällen (z. B. generalisierten tonisch-klonischen Anfällen, asymmetrischen tonischen Anfällen), Leukodystrophie, Leukoenzephalopathie, geistiger Behinderung, multifokaler Epilepsie, medikamentenresistenter Epilepsie, Temporallappenepilepsie oder zerebellärer Ataxie) ausgewählt ist.

12. Verbindung oder pharmazeutisch unbedenkliches Salz davon oder pharmazeutische Zusammensetzung für die Verwendung nach Anspruch 9, wobei die neurologische Erkrankung, die Erkrankung, die mit übermäßiger neuronaler Erregbarkeit assoziiert ist, oder die Erkrankung, die mit einer Funktionsgewinnmutation eines Gens (z. B. KCNT1) assoziiert ist, aus einer Herzfunktionsstörung, Herzrhythmusstörung, Brugada-Syndrom, Myokardinfarkt, Schmerzen und damit verbundenen Zuständen (z. B. neuropathischen Schmerzen, akuten/chronischen Schmerzen, Migräne), einer Muskelerkrankung (z. B. Myotonie, Neuromyotonie, Krampfmuskelkrämpfen, Spastik), Juckreiz und Pruritus, Ataxie, zerebellären Ataxien, psychiatrischen Erkrankungen (z. B. schwerer Depression, Angstzuständen, bipolarer Erkrankung, Schizophrenie), einer Lernstörung, Fragile-X-Syndrom, neuronaler Plastizität oder einer Autismus-Spektrum-Erkrankung ausgewählt ist.

13. Verbindung oder pharmazeutisch unbedenkliches Salz davon oder pharmazeutische Zusammensetzung für die Verwendung nach Anspruch 9, wobei die neurologische Erkrankung, die Erkrankung, die mit übermäßiger neuronaler Erregbarkeit assoziiert ist, oder die Erkrankung, die mit einer Funktionsgewinnmutation eines Gens (z. B. KCNT1) assoziiert ist, aus epileptischer Enzephalopathie mit SCN1A-, SCN2A- und/oder SCN8A-Mutationen, frühkindlicher epileptischer Enzephalopathie, Dravet-Syndrom, Dravet-Syndrom mit SCN1A-Mutation, generalisierter Epilepsie mit Fieberkrämpfen, schwer behandelbarer Epilepsie im Kindesalter mit generalisierten tonisch-klonischen Anfällen, infantilen Krämpfen, gutartigen familiären neonatalen-infantilen Anfälle, SCN2A-epileptischer Enzephalopathie, fokaler Epilepsie mit SCN3A-Mutation, kryptogener pädiatrischer partieller Epilepsie mit SCN3A-Mutation, SCN8A-epileptischer Enzephalopathie, Rasmussen-Enzephalitis, malignen migrierenden partiellen Anfällen im Säuglingsalter, autosomal-dominanter nächtlicher Frontallappenepilepsie, KCNQ2-epileptischer Enzephalopathie oder KCNT1-epileptischer Enzephalopathie ausgewählt ist.

## Revendications

1. Composé de formule I présentant un noyau oxadiazole : 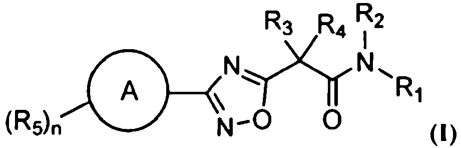 ou sel pharmaceutiquement acceptable de celui-ci, dans lequel
le cycle A est un hétéroaryle de 5 ou 6 chaînons,
R₁ est choisi parmi un alkyle en C₁₋₆, cycloalkyle en C₃₋₈, phényle, hétérocyclyle de 3 à 7 chaînons ou hétéroaryle de 5 à 6 chaînons, lesdits alkyle en C₁₋₆, cycloalkyle en C₃₋₈, phényle, hétérocyclyle de 3 à 7 chaînons ou hétéroaryle de 5 ou 6 chaînons comprenant éventuellement un ou plusieurs substituants R₆,
R₂ est choisi parmi l'hydrogène ou un alkyle en C₁₋₆, ou
R₁ et R₂ sont pris conjointement avec l'azote lié à R₁ et R₂ pour former un hétérocyclyle de 3 à 7 chaînons comprenant éventuellement un ou plusieurs substituants R₆,
R₃ est choisi parmi l'hydrogène ou un alkyle en C₁₋₆ comprenant éventuellement un ou plusieurs substituants choisis parmi un halogène, halogénoalkyle en C₁₋₆, alcoxy en C₁₋₆ ou halogénoalcoxy en C₁₋₆,
R₄ est un alkyle en C₁₋₆ comprenant éventuellement un ou plusieurs substituants choisis parmi un halogène, halogénoalkyle en C₁₋₆, alcoxy en C₁₋₆ ou halogénoalcoxy en C₁₋₆, ou
R₃ et R₄ sont pris conjointement avec le carbone lié à R₃ et R₄ pour former un cycloalkylène en C₃₋₈ ou hétérocycloalkylène de 3 à 7 chaînons,
chaque R₅ est indépendamment choisi parmi un halogène, alkyle en C₁₋₆, halogénoalkyle en C₁₋₆, alcoxy en C₁₋₆, halogénoalcoxy en C₁₋₆, -N(R₇)₂, (alkylène en C₁₋₆)-(alcoxy en C₁₋₆) ou cycloalkyle en C₃₋₈,
chaque R₆ est indépendamment choisi parmi un halogène, alkyle en C₁₋₆, halogénoalkyle en C₁₋₆, alcoxy en C₁₋₆, halogénoalcoxy en C₁₋₆, cycloalkyle en C₃₋₈, hétérocyclyle de 3 à 7 chaînons, hétéroaryle de 5 à 6 chaînons ou phényle,
chacun des R₇ est indépendamment choisi parmi un hydrogène, alkyle en C₁₋₆ ou - (alkylène en C₁₋₆)-OH, ou les deux R₇ sont pris conjointement avec l'atome d'azote lié aux deux R₇ pour former un hétérocycle comprenant éventuellement un ou plusieurs substituants choisis parmi un halogène et -OH, et
n vaut 0, 1, 2 ou 3.

2. Composé selon la revendication 1, ledit composé étant un composé de formule I-a : 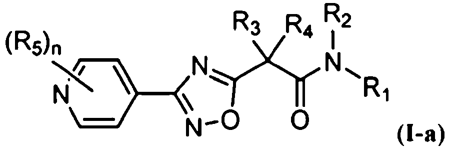 ou sel pharmaceutiquement acceptable de celui-ci.

3. Composé selon la revendication 1 ou 2, ledit composé étant un composé de formule I-**a1**, formule **I-a2,** formule **I-a3,** formule **I-a4** ou formule **I-a5** : 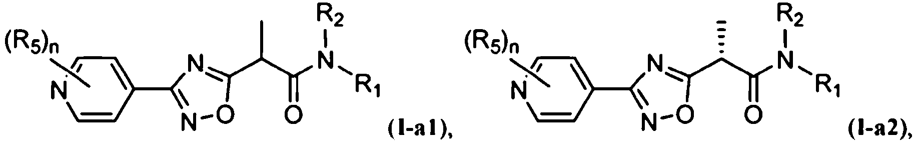 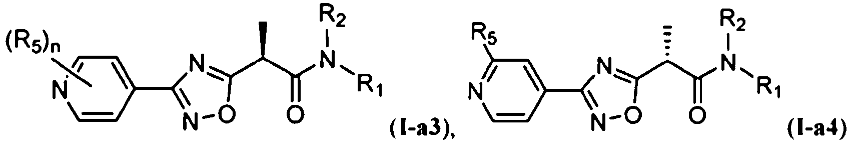 ou 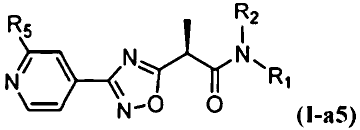 ou sel pharmaceutiquement acceptable de celui-ci.

4. Composé selon l'une quelconque des revendications 1 à 3, dans lequel R₁ est choisi parmi un alkyle en C₁₋₆, cycloalkyle en C₃₋₈ ou phényle, chacun comprenant éventuellement un substituant R₆ ; R₁ étant de préférence un alkyle en C₁₋₆ comprenant un substituant R₆, tel qu'un substituant phényle.

5. Composé selon l'une quelconque des revendications 1 à 4, dans lequel R₂ représente un hydrogène.

6. Composé selon l'une quelconque des revendications 1, 2, et 4 et 5, dans lequel R₃ représente un alkyle en C₁₋₆, tel qu'un méthyle.

7. Composé selon la revendication 1, ledit composé étant choisi parmi : 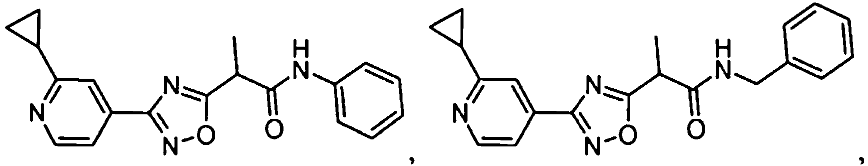 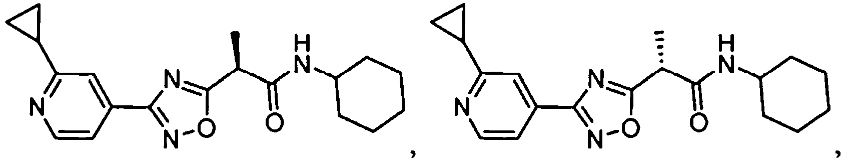 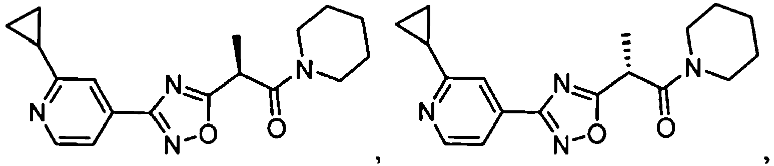 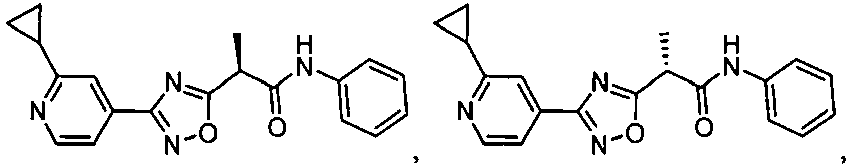 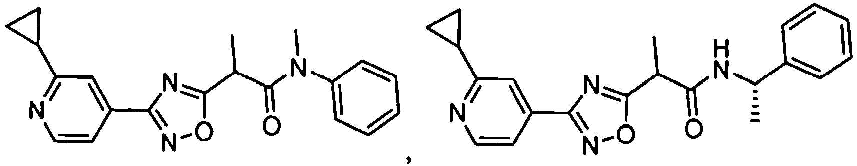 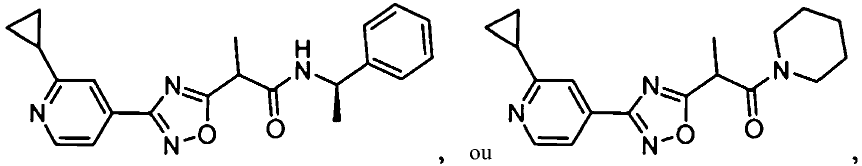 ou sel pharmaceutiquement acceptable de celui-ci.

8. Composition pharmaceutique comprenant un composé selon l'une quelconque des revendications 1 à 7, ou un sel pharmaceutiquement acceptable de celui-ci, et un excipient pharmaceutiquement acceptable.

9. Composé selon l'une quelconque des revendications 1 à 7 ou sel pharmaceutiquement acceptable de celui-ci ou composition pharmaceutique selon la revendication 8, destinés à une utilisation dans une méthode de traitement d'un trouble neurologique, d'un trouble associé à une excitabilité neuronale excessive ou d'un trouble associé à une mutation de gain de fonction d'un gène (par exemple KCNT1).

10. Composé ou sel pharmaceutiquement acceptable de celui-ci ou composition pharmaceutique destinés à une utilisation selon la revendication 9, ledit trouble neurologique, trouble associé à une excitabilité neuronale excessive ou trouble associé à une mutation de gain de fonction d'un gène (par exemple KCNT1) étant l'épilepsie, un syndrome épileptique, une encéphalopathie, tels qu'une épilepsie génétique ou pédiatrique ou un syndrome épileptique génétique ou pédiatrique.

11. Composé ou sel pharmaceutiquement acceptable de celui-ci ou composition pharmaceutique destinés à une utilisation selon la revendication 9, ledit trouble neurologique, trouble associé à une excitabilité neuronale excessive ou trouble associé à une mutation de gain de fonction d'un gène (par exemple KCNT1) étant choisi parmi : l'épilepsie et autres encéphalopathies (par exemple crises focales migrantes malignes du nourrisson (MMFSI) ou épilepsie du nourrisson avec crises focales migrantes (EIMFS), épilepsie frontale nocturne familiale autosomique dominante (ADNFLE), syndrome de West, spasmes infantiles, encéphalopathie épileptique, épilepsie focale, syndrome d'Ohtahara, encéphalopathie épileptique et développementale, syndrome de Lennox-Gastaut, crises (par exemple crises tonicocloniques généralisées, crises toniques asymétriques), leucodystrophie, leucoencéphalopathie, déficience intellectuelle, épilepsie multifocale, épilepsie pharmacorésistante, épilepsie temporale ou ataxie cérébelleuse).

12. Composé ou sel pharmaceutiquement acceptable de celui-ci ou composition pharmaceutique destinés à une utilisation selon la revendication 9, ledit trouble neurologique, trouble associé à une excitabilité neuronale excessive ou trouble associé à une mutation de gain de fonction d'un gène (par exemple KCNT1) étant choisi parmi : dysfonction cardiaque, arythmie cardiaque, syndrome de Brugada, infarctus du myocarde, douleur et affections associées (par exemple douleur neuropathique, douleur aiguë/chronique, migraine), un trouble musculaire (par exemple myotonie, neuromyotonie, crampes musculaires, spasticité), démangeaisons et prurit, ataxie, ataxies cérébelleuses, troubles psychiatriques (par exemple dépression majeure, anxiété, trouble bipolaire, schizophrénie), un trouble de l'apprentissage, le syndrome de l'X fragile, la plasticité neuronale ou un trouble du spectre autistique.

13. Composé ou sel pharmaceutiquement acceptable de celui-ci ou composition pharmaceutique destinés à une utilisation selon la revendication 9, ledit trouble neurologique, trouble associé à une excitabilité neuronale excessive ou trouble associé à une mutation de gain de fonction d'un gène (par exemple KCNT1) étant choisi parmi : encéphalopathie épileptique avec mutations sur SCN1A, SCN2A et/ou SCN8A, encéphalopathie épileptique infantile précoce, syndrome de Dravet, syndrome de Dravet avec mutation sur SCN1A, épilepsie généralisée avec crises fébriles, épilepsie infantile réfractaire avec crises tonicocloniques généralisées, spasmes infantiles, crises néonatales-infantiles familiales bénignes, encéphalopathie épileptique SCN2A, épilepsie focale avec mutation sur SCN3A, épilepsie partielle pédiatrique cryptogénique avec mutation sur SCN3A, encéphalopathie épileptique SCN8A, encéphalite de Rasmussen, crises partielles migrantes malignes du nourrisson, épilepsie frontale nocturne familiale autosomique dominante, encéphalopathie épileptique KCNQ2 ou encéphalopathie épileptique KCNT1.
